# EUROPEAN PATENT SPECIFICATION

(11) **EP 2 205 739 B1**
(45) Date of publication and mention of the grant of the patent: **02.07.2014**
(21) Application number: 08806365.6
(22) Date of filing: 22.09.2008
(51) Int. Cl.: C12N 15/11

(54) **TARGETED MODULATION OF GENE EXPRESSION**
GEZIELTE MODULATION DER GENEXPRESSION
MODULATION CIBLÉE DE L'EXPRESSION GÉNIQUE

(30) Priority: 22.09.2007 GB 0718542
(43) Date of publication of application: 14.07.2010
(73) Proprietor: University Court of The University of Dundee, Dundee DD1 4HN (GB)
(72) Inventor: LAMOND, Angus, Lain, Dundee DD1 5EH (GB); ONO, Motoharu, Dundee DD1 5EH (GB)
(74) Representative: Chapman, Paul Gilmour
(86) International application number: PCT/GB2008/003211
(87) International publication number: WO 2009/037490

(56) References cited:
- HENRAS ANTHONY K ET AL: "RNA structure and function in C/D and H/ACA s(no)RNPs" CURRENT OPINION IN STRUCTURAL BIOLOGY, vol. 14, no. 3, June 2004 (2004-06), pages 335-343, XP002510433 ISSN: 0959-440X
- REICHOW STEVE L ET AL: "The structure and function of small nucleolar ribonucleoproteins" NUCLEIC ACIDS RESEARCH, vol. 35, no. 5, 6 February 2007 (2007-02-06), pages 1452-1464, XP002510434 ISSN: 0305-1048
- ONO M ET AL: "Targetes gene regulation mediated by nucleolar snoRNAs" FEBS JOURNAL, vol. 275, no. Suppl. 1, June 2008 (2008-06), page 73, XP009110780 & JOINT CONFERENCE OF THE 33RD FEBS CONGRESS/11TH IUBMB CONFERENCE; ATHENS, GREECE; JUNE 28 -JULY 03, 2008 ISSN: 1742-464X(print) 1742-4658(ele
- ENDER CHRISTINE ET AL: "A Human snoRNA with MicroRNA-Like Functions" MOLECULAR CELL, vol. 32, no. 4, November 2008 (2008-11), pages 519-528, XP002510436 ISSN: 1097-2765

## Description

### Field of the Invention

The present invention relates to a method of modulating gene expression using snoRNA molecules or snoRNA like molecules or fragments, designed to target specific nucleic acid sequences.

The present invention also relates to modified snoRNA molecules and modified snoRNA like molecules and fragments for use in targeted modulation of gene expression.

### Background of the Invention

Small nucleolar RNAs (snoRNAs) comprise a family of nuclear RNAs that are present in all eukaryotic cells. snoRNAs are concentrated in the nucleus, particularly in nucleoli where they function in either the modification of ribosomal RNA (rRNA) or else participate in the processing of rRNA during ribosome subunit synthesis. There are two main classes of snoRNAs, the box C/D snoRNAs and the box H/ACA snoRNAs, which are involved in 2'-O-ribose methylation and psuedouridine modifications, respectively (reviewed in Boisvert et. al., 2007, Kiss et al., 2002 and 2006)

SnoRNAs function in vivo as RNA-protein complexes (snoRNPs), where the RNA moiety acts as a guide RNA that base pairs to rRNA precursors, either to direct sites of modification to specific sequences on rRNA (in the case of box C/D and box H/ACA snoRNAs) or as chaperones to assist the maturation of nascent rRNA precursors (e.g. U3 snoRNA).

Box C/D snoRNAs are named after a common RNA motif in this subfamily that serves as a binding site for a group of box C/D proteins, including NOP56, NOP58, TIP48, TIP49, 15.5K and the highly conserved protein fibrillarin, which has the specific 2-O-methylase activity. A region of the box C/D snoRNA immediately 5' to the stem II & box D region contains the 'guide' sequence complementary to a specific site on rRNA that directs the fibrillarin 2'-O-methylase to add a methyl group at the 2'-OH position of the desired ribose residue within the rRNA sequence that is complementary to the guide RNA region. A similar function has been shown to operate from yeast through to mammalian cells. Because of the conserved sequence elements it is possible to use genomic DNA sequence information to predict members of the human box C/D snoRNA family and predict their putative 2'-O-methylation target sites on rRNA through computational analysis. However, so far in the case of human not all of these species have been identified or verified by direct experimentation.

In addition to the modification of rRNA, two other functions have been proposed for certain box C/D snoRNAs. First, several family members are thought to specify methylation of 2'-O-ribose groups on other non-ribosomal RNAs, including small nuclear RNAs, which are subunits of the nuclear pre-mRNA splicing machinery. This is basically the same modification reaction as seen for rRNA, but directed to different RNA substrates due to the guide RNA sequences they contain. Second, one member of the box C/D snoRNA family (HBII-52) (S. Kishore and S. Stamm, 2006), has been reported to act as an alternative splicing factor that can cause a change in the choice of splice site, affecting Exon 5 in the 6 Exon neuronal-specific transcript encoding Serotonin receptor 2C. In this case the mechanism involved is not known and it is also not clear whether the HBII-52 snoRNA is also involved in modification of rRNA or whether it is localised to the nucleolus.

Being able to control gene expression has been studied for many years and a variety of techniques are known, all with advantages and disadvantages. RNAi techniques, for example, are well known in the art. "RNAi" refers to the process of sequence-specific post transcriptional gene silencing in animals and plants, initiated by double-stranded RNA, that is identical to a region of target RNA and leads to its degradation (see for example WO9932619, WO0129058 and Tuschl Chem Biochem. 2: 239-245 (2001)). However, there are problems associated with RNAi techniques, not least the cost when one has to synthesise the molecules using chemical means. Moreover, when making RNAi molecules using genetic approaches, it can be difficult to generate the molecules appropriately. Also, to produce more than one RNAi molecule to target the same or differing target sequence, simply compounds the aforementioned problems. Problems with RNAi can also arise in achieving high efficiency knockdown of targeted genes, especially without causing unwanted "off-target" knockdown of additional genes.

Thus further or alternative methods of gene silencing, or modulation of expression would be desirable. It is an object of the present invention to obviate and/or mitigate at least one of the aforementioned disadvantages.

### Summary of the Invention

The present invention is based in part on studies by the present inventors into snoRNAs and the identification that a portion of a snoRNA sequence can be replaced with a target specifc nucleic acid sequence such that a modified snoRNA is formed, which when added to a cell is, or a fragment thereof is, capable of modulating expression of a gene comprising the target specific nucleic acid sequence.

In a first aspect there is provided a method of modulating expression of a target nucleic acid, the method comprising contacting the nucleic acid with a snoRNA under conditions whereby the snoRNA and/or fragment thereof is capable of hybridising to a portion of the target nucleic acid; and wherein hybridisation of the snoRNA or fragment thereof to said portion of nucleic acid either modulates or causes modulation of expression and/or function of the target nucleic acid. The snoRNA may be a native snoRNA molecule, or it may be a modified snoRNA molecule as described further hereinafter.

There is also provided a modified snoRNA sequence for use in modulating expression of a target nucleic acid, the modified snoRNA sequence comprising a nucleic acid sequence substantially complementary to a portion of a target nucleic acid sequence, for hybridising to said portion of the target nucleic acid and modulating expression of the nucleic acid. It is to be understood, that such modified snoRNA molecules are not identical to native snoRNA molecules known in the art, such as the molecule HBII-180C. Generally speaking such modified snoRNA molecules are based on native snoRNA molecules, as discussed below, but comprise a portion of nucleic acid specifically selected and introduced into the snoRNA molecule, so as to hybridise to a portion of target nucleic acid and modulate its expression.

The inventors have observed that the snoRNA molecules according to the present invention are capable of down-regulating or reducing expression/function of a target RNA or protein. Without wishing to be bound by theory, it is possible that the modified snoRNA may be processed within a cell, such that a fragment of the snoRNA molecule, which comprises a sequence capable of specifically hydridising to the target sequence, may have the effect of modulating expression/function of the target RNA, although it is also possible that expression may be modulated through binding to DNA at particular loci. Such a sequence may correspond exactly, or to a high degree of specificity with the sequence present in the native and modified snoRNAs as described herein, which is substantially complementary to said portion of target nucleic acid sequences, such as RNA or DNA sequence, or may be shorter in length, depending upon the base composition and G/C content of the sequence, such as by 2-10 nucleotides. In principle the complementary region will be of sufficient length and sequence as to provide specific binding to the target RNA through known principles of complementary base pairing.

The snoRNA molecules of the present invention may be based on so-called box C/D-snoRNA or box H/ACA-snoRNA. Preferred snoRNAs are based on box C/D-snoRNA.

As used herein, the phrase "snoRNA" refers to small RNA molecules which usually are synthesized and/or function in the nucleoplasm and/or the nucleolus of the cell. According to the preferred embodiments the small nuclear RNA molecules of the present invention are snoRNAs which contain the box C/D.

Non-limiting examples of box C/D snoRNAs include the *L. collosoma* b2 (GenBank Accession No. AF331656), *L. collosoma* B3 (GenBank Accession No. AY046598), *L. collosoma* B4 (GenBank Accession No. AY046598), *L. collosoma* B5 (GenBank Accession No. AY046598), *L. collosoma* TS1 (GenBank Accession No. AF331656), *L. collosoma* TS2 (GenBank Accession No. AF331656), *L. collosoma* g2 (GenBank Accession No. AF331656), *L. collosoma* snoRNA-2 (GenBank Accession No. AF050095), *T. brucei* snoRNA 92 (GenBank Accession No. Z50171, *L. tarentolae* snoRNA 92 (GenBank Accession No. AF016399), *T. brucei* TBC4 snoRNA (SEQ ID NO:35), *T. brucei* sno 270 (GenBank Accession No. Z50171) and human U14 snoRNA (GenBank Accession No. NR_000022).

The modified snoRNAs of the present invention may comprise one or more D/D' box nucleic acid sequences commonly found in the box C/D snoRNAs. The D and/or D' box is a conserved sequence of nucleotides and can consist of a sequence selected from, for example, 5'-CUGA-3', 5'-AUGA-3', 5'-CCGA-3', 5'-CAGA-3', 5'-CUUA-3', 5'-UUGG-3' and 5'-CAGC-3'. However, further modifications and/or derivatives may be envisaged.

The modified snoRNAs of the present invention may further comprise a sequence complementary to 28S rRNA and/or a Box C sequence. The sequence complementary to 28S rRNA may be from 5-15 nucleotides in length, such as 8 - 12 nucleotides, especially 10 nucleotides, but this rRNA complementary region can also be mutated and the complentarity to rRNA substantially or entirely removed without preventing activity. Typically the sequence complementary to 28S rRNA may be complementary to nucleotides at or around base 3680 of the 28S rRNA sequence (numbering according to Lestrade, L., and Weber, M. J. (2006). snoRNA-LBME-db, a comprehensive database of human H/ACA and C/D box snoRNAs. Nucleic Acids Res 34, D158-162.), such as around 3670 - 3690, e.g. 3677 - 3686. The box C sequence is typically 5 -9 nucleotides in length, such as 7 nucleotides and may comprise the sequence AUGAUGU or a portion thereof. Typically when present a box C sequence is located 5' of a sequence complementary either to rRNA, or to other physiological RNA targets of snoRNA, which is 5' of a box D' sequence, which box D' sequence is 5' to the nucleic acid sequence which is substantially complementary to said portion of the target nucleic acid sequence. Preferably only 1 - 3, such as 1 nucleotide base is found between the box C sequence, sequence complementary to rRNA, box D' sequence and/or the nucleic acid sequence which is substantially complementary to said portion of target nucleic acid sequence. A box D sequence identical or otherwise to the box D' sequence may be found 3' of the nucleic acid sequence which is substantially complementary to said portion of target RNA sequence. The box D sequence may be located 20 - 30 nucleotides 3' of the 3' base of the nucleic acid sequence which is substantially complementary to said portion of target nucleic acid sequence, such as 24 - 28 nucleotides, especially 26 nucleotides downstream.

The modified snoRNA molecules of the present invention also comprise at least one sequence capable of targeting and hybridising to a target nucleic acid sequence. Based on the work presented herein, the inventors identified a snoRNA molecule, HBII-180C, which comprised a sequence immediately downstream from the Box D' sequence which was highly complementary to a 21nt sequence found within intron 17 of the pre-mRNA transcribed from human fibroblast growth factor 3 (FGFR3). By changing this sequence to a different sequence designed to be complementary to a particular target gene sequence, which may be an intron or exon sequence the present inventors have been able to modulate expression of desired target genes.

Typically the nucleic acid sequence, which is substantially complementary to said portion of target nucleic acid sequence is 15-45 nucleotides in length, such as 17-30 nucleotides, but may be longer depending upon sequence and base composition. By "substantially" complementary means that there does not need to be exact complementarity between the target nucleic acid sequence and the sequence of the snoRNA molecule designed to hybridise to the target nucleic acid. However, it is to be understood that there should be a high degree of identify, typically greater than 90 or 95%, which is sufficient to ensure specific binding according to known RNA or DNA/RNA base pairing rules. Thus, typically at most only 1 - 3 mismatches between the two sequences may be tolerated, depending upon length and G/C content of the complementary region. The target nucleic acid sequence may be an RNA sequence, typically an mRNA, tRNA, miRNA or rRNA sequence, or an RNA virus genomic sequence or transript derived thereof, especially an mRNA sequence. Within a target nucleic acid sequence the complementary region can be either in the intron, or exon, or particularly at intron - exon junctions, but can also reside in 5' or 3' untranslated regions.

It is to be appreciated that more than one sequence designed to hybridise to a target nucleic acid molecule may be found within a modified snoRNA molecule of the present invention. When there is more than one such sequence, the different "targeting" sequences may be designed to target different portions of the same target nucleic acid molecule, or different target nucleic acid molecules.

In a further aspect, there is also provided a nucleic acid construct capable of expressing at least one snoRNA molecule according to the present invention.

Typically the nucleic acid construct comprises at least two regions of exonic nucleic acid flanking a region of intronic nucleic acid, which is capable of encoding a snoRNA according to the present invention. Most desirably the nucleic acid construct may comprise a multiplicity of exonic sequences flanking two or more intronic sequences comprising sequence capable of encoding one or more snoRNAs according to the present invention. Such a construct may be formed as a single construct, which upon transcription within a target cell leads to generation of an mRNA corresponding to the exonic sequences and splicing out of the intronic sequence and subsequent generation of a snoRNA(s) according to the present invention.

Where more than one intronic sequence is employed to generate more than one snoRNA sequence of the present invention, each snoRNA may be designed to target the same or different target RNA molecules.

It is expected that the skilled addressee is well familiar with what constitutes an intron and exon, but for assistance the following is provided:

Introns are portions of eukaryotic DNA which intervene between the portions, or "exons", of that DNA which are the regions that are expressed in the final mRNA product, usually coding for protein. Introns and exons are transcribed into RNA termed "primary transcript, precursor to mRNA" (or "pre-mRNA"). Introns must be removed from the pre-mRNA so that the native protein encoded by the exons can be produced (the term "native protein" as used herein refers to naturally occurring, wild type, mutant or functional protein). The removal of introns from pre-mRNA and subsequent joining of the exons is carried out in the splicing process.

The splicing process is actually a series of reactions, mediated by splicing factors, which is carried out on RNA after transcription but before translation. Thus, a "pre-mRNA" is an RNA which contains both exons and intron(s), and an "mRNA" is an RNA in which the intron(s) have been removed and the exons joined together sequentially so that the protein may be translated therefrom by the ribosomes.

Introns are defined by a set of "splice elements" which are relatively short, conserved RNA segments which bind the various splicing factors which carry out the splicing reactions. Thus, each intron is defined by a 5' splice site, a 3' splice site, and a branch point situated therebetween.

The nucleic acid construct may further comprise common nucleic acid features often found in conventional nucleic acid vectors and well known to the skilled addressee. For example, the nucleic acid construct may comprise a selection marker gene for facilitating identification of cells into which the nucleic acid construct has been transformed or transfected. Preferably, the nucleic acid construct comprises at least one promoter, such as a constitutive or controllable promoter known in the art, for facilitating expression of the nucleic acid encoding said snoRNA molecule(s). Examples of suitable promoters include the CMV promoter, T3, T7, SP6, SV40, adenovirus major late promoter and others known to the skilled addressee.

The nucleic acid construct may also comprise a copy of the gene/RNA in a native, mutant or otherwise tagged form, which is designed to replace the copy of the gene/RNA being modulated by said snoRNA.

Several approaches can be used to produce the snoRNA of the present invention in a cell.

According to one preferred embodiment of the present invention the snoRNA molecule of the present invention can be produced by introducing into a cell, a nucleic acid construct capable of expressing the snoRNA molecule described above.

In order to express the snoRNA of the present invention in the cell a nucleic acid sequence is ligated into a nucleic acid construct, which includes a promoter upstream of a nucleic acid sequence encoding a snoRNA. Desirably the sequence encoding a snoRNA is flanked on either side by regions of nucleic acid identifiable by the skilled addressee as exon sequences and splicing sequences, which lead to splicing out of the sequence encoding the snoRNA upon transcription. Promoters which are suitable for directing the transcription of the nucleic acid sequence in, for example, eukaryotic cells include constitutive or inducible promoters.

Constitutive promoters suitable for use with the present invention are promoter sequences, which are active under most environmental conditions and most types of cells such as the CMV promoter, SV40 promoter, adenovirus major late promoter and Rous sarcoma virus (RSV) promoter. Inducible promoters suitable for use with the present invention include for example the hypoxia-inducible factor 1 (HIF-1) promoter (Rapisarda, A. et al., 2002. Cancer Res. 62: 4316-24) and the tetracycline-inducible promoter (Srour, M.A., et al., 2003. Thromb. Haemost. 90: 398-495).

A nucleic acid construct of the present invention generally includes additional sequences which render the construct suitable for replication and/or integration in prokaryotes, eukaryotes, or preferably both (e.g. shuttle vectors). Typical cloning vectors contain transcription and translation initiation sequences (e.g., promoters, enhancers) and transcription and translation terminators (e.g., polyadenylation signals).

In the construction of the nucleic acid construct, the promoter(s) is preferably positioned at approximately the same distance from the heterologous transcription start site as it is from the transcription start site in its natural setting. As is known in the art, however, some variation in this distance can be accommodated without loss of promoter function.

In addition to the elements already described, the nucleic acid construct of the present invention may typically contain other specialised elements intended to increase the level of expression of cloned nucleic acids or to facilitate the identification of cells that carry the recombinant DNA. For example, a number of animals viruses contain DNA sequences that promote the extra chromosomal replication of the viral genome in permissive cell types. Plasmids bearing these viral replicons are replicated episomally as long as the appropriate factors are provided by genes either carried on the plasmid or with the genome of the host cell.

The nucleic acid construct may or may not include a eukaryotic replicon. If a eukaryotic replicon is present, then the vector is amplifiable in eukaryotic cells using the appropriate selectable marker. If the vector does not comprise a eukaryotic replicon, no episomal amplification is possible. Instead, the recombinant DNA integrates into the genome of the engineered cells, where the promoter directs expression of the desired nucleic acid. Such contructs may also comprise site-specific recombination sites, designed to target the nucleic acid construct to a specific site in a cell's genome and to integrate at the specific site when the necessary enzymes are present in the cell. A variety of site-specific recombination systems are well known to those skilled in the art, including Cre/Lox, Att/λintegrase, frt/Flp, gamma delta resolvase, Tn3 resolvase and ØC231 integerase (see Gover et al., 2005, to which the skilled reader is directed).

The nucleic acid construct of the present invention can be used to express the polynucleotide of the present invention in mammalian cells (e.g., HeLa cells, Cos cells), yeast cells (e.g., AH109, HHY10, KDY80), insect cells (e.g., Sf9), trypanosome cells (e.g., *L. collosomm L. major, T.brucei* 29-13) or bacteria cells (e.g., JM109, RP437, MM509, SW10).

Preferably, the polynucleotide of the present invention is synthesised by ligating a nucleic acid sequence into a mammalian, yeast, trypanosome or bacterial expression vector. Examples of such vectors include but are not limited to the pcDNA3.1, pBK-CMB and pCI vectors which are suitable for use in mammalian cells, the pGBKT7, pLGADH2-lacZ and pBGM18 vectors which are suitable for use in yeast cells, the pX-*neo* episomal vector which is suitable for use in trypanosome cells and the Pack02scKan, pMLBAD, pMLS7 vectors which are suitable for use in bacterial cells.

According to preferred embodiments the nucleic acid construct of the present invention is preferably constructed for eukaryotic expression, most preferably, mammalian cell expression.

Examples of mammalian expression vectors include, but are not limited to, pcDNA3, pcDNA3.1(+/-), Pgl3, PzEOsv2(+/-), pSecTag2, pDisplay, pEF/myc/cyto, pCMV/myc/cyto, pCR3.1, pSinRep5, DH26S, DHBB, pNMT1, pNMT41, pNMT81, which are available from Invitrogen, pCI which is available from Promega, pMbac, pPbac, pBK-RSV and pBK-CMV which are available from Stratagene, pTRES which is available from Clontech, and their derivatives.

Expression vectors containing regulatory elements from eukaryotic viruses such as retroviruses can be also used. SV40 vectors include pSVT7 and pMT2. Vectors derived from bovine papilloma virus include pBV-1MTHA, and vectors derived from Epstein Bar virus include pHEBO, and p205. Other exemplary vectors include pMSG, pAV009/A⁺, pMTO10/A⁺, pMAMneo-5, baculovirus pDSVE, and any other vector allowing expression of proteins under the direction of the SV-40 early promoter, SV-40 later promoter, metallothionein promoter, murine mammary tumour virus promoter, Rous sarcoma virus promoter, polyhedron promoter, or other promoters shown effective for expression in eukaryotic cells.

Various methods can be used to introduce the nucleic acid construct of the present invention into mammalian cells. Such methods are generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, An Arbor, Mich. (1995), Vega et al., Gene Targeting, CRC Press, Ann Arbor Mich. (1995), Vectors: A Survey of Molecular Cloning Vectors and Their Uses, Butterworths, Boston Mass. (1988) and Gilboa et al. [Biotechniques 4 (6): 504-512, 1986] and include, for example, stable or transient transfection, lipofection, electroporation, microinjection, liposomes, iontophoresis, receptor-mediated endocytosis and infection with recombinant viral vectors. In addition, see U.S. Pat. Nos. 5,464,764 and 5,487,992 for positive-negative selection methods. For example, for stable transfection in dihydrofolate reductase deficient Chinese Hamster Ovary (CHO dhfr-) cells the expression vector of the present invention further includes a dihydrofolate reductase expression cassette positioned under a control of a thymidine kinase promoter.

The nucleic acid construct of the present invention may also be delivered into a cell using viral vectors, such as lentivinis, retrovirus or adenovirus derived vectors known in the art.

In a further aspect of the present invention, there is provided a nucleic acid vector construct for use in generating a snoRNA molecule according to the present invention, the construct comprising in a 5' to 3' direction
i) a promoter sequence for controlling transcription;
ii) a first exon sequence;
iii) a first intron splicing sequence;
iv) a cloning site or sequence for facilitating cloning of a nucleic acid sequence encoding a snoRNA of the present invention;
v) a second intron splicing sequence; and
vi) a second exon sequence.
The snoRNA may be a native or modified snoRNA molecule as described herein.

It is to be appreciated that the various components are transcriptionally linked as would be understood by the skilled addressee. The vector may naturally comprise other components as described herein above, and may include additional cloning sites to facilitate vector construction.

One particular advantage of the present invention is the ability to provide multiple snoRNAs from a single transcript, which can target the same or different target nucleic acid sequences.

As an alternative to the use of nucleic acid constructs, it will be appreciated that the snoRNA molecules of the present invention can be chemically synthesised using for example, solid phase synthesis, as an RNA oligonucleotide.

Several considerations must be taken into account when designing synthetic snoRNA molecules, snoRNA like molecules or fragments thereof of the present invention. For efficient *in vivo* inhibition of gene expression the molecules may desirable fulfil the following requirements (i) sufficient specificity in binding to the target sequence; (ii) solubility in water; (iii) stability against intra- and extracellular nucleases; (iv) capability of penetration through the cell membrane; and (v) when used to treat an organism, low toxicity.

Unmodified polynucleotides may be impractical for use since they have short *in vivo* half-lives, during which they can be degraded rapidly by nucleases. Furthermore, they are difficult to prepare in more than milligram quantities. In addition, such polynucleotides are poor cell membrane penetrants.

In order to improve half-life as well as membrane penetration, the polynucleotide backbone of the polynucleotide of the present invention can be modified.

Polynucleotides can be modified either in the base, the sugar or the phosphate moiety. These modifications include, for example, the use of methylphosphonates, monothiophosphates, dithiophosphates, phosphoramidates, phosphate esters, bridged phosphorothioates, bridged phosphoramidates, bridged methylenephosphonates, dephospho internucleotide analogs with siloxane bridges, carbonate bridges, carboxymethyl ester bridges, carbonate bridges, carboxymethyl ester bridges, acetamide bridges, carbamate bridges, thioether bridges, sulfoxy bridges, sulfono bridges, anomeric bridges and borane derivatives (Cook, 1991, Medicinal chemistry of antisense oligonucleotides-future opportunities. Anti-Cancer Drug Design 6: 585). Preferably, to render an *in vivo* stability to the synthetic polynucleotide of the present invention, the oxygen molecule at position 2 of the ribose ring may be methylated, resulting in 2'-O-methylated RNA oligonucleotides.

International patent application WO 89/12060 discloses various building blocks for synthesising polynucleotide analogs, as well as polynucleotide analogs formed by joining such building blocks in a defined sequence. The building blocks may be either "rigid" (i.e., containing a ring structure) or "flexible" (i.e., lacking a ring structure). In both cases, the building blocks contain a hydroxy group and a mercapto group, through which the building blocks are said to join to form polynucleotide analogs. The linking moiety in the oligonucleotide analogs is selected from the group consisting of sulphide (-S-), sulfoxide (-SO-), and sulfone (-SO2-).

International patent application WO 92/20702 describe an acyclic oligonucleotide which includes a peptide backbone on which any selected chemical nucleobases or analogs are strunged and serve as coding characters as they do in natural RNA. These new compounds, known as peptide nucleic acids (PNAs), are not only more stable in cells than their natural counterparts, but also bind the natural RNA 50 to 100 times more tightly than the natural nucleic acids cling to each other. PNA oligomers can be synthesised from the four protected monomers containing uridine, cytosine, adenuine and guanine by Merrifield solid-phase peptide synthesis. In order to increase solubility in water to prevent aggregation, a lysine amide group is placed at the C-terminal region.

snoRNA stability can also be increased by incorporating 3'-deoxythymidine or 2'-substituted nucleotides (substituted with, e.g., alkyl groups) into the snoRNAs during synthesis, by providing the snoRNAs as phenylisourea derivatives, or by having other molecules, such as aminoacridine or polylysine, linked to the 3' ends of the snoRNAs (see, e.g., Anticancer Research 10:1169-1182, 1990). Modifications of the RNA nucleotides of the snoRNAs of the invention may be present throughout the snoRNA, or in selected regions, e.g., the 5' and/or 3' ends. The snoRNAs can also be modified to increase their ability to penetrate the target tissue by, e.g., coupling them to lipophilic compounds. The snoRNAs of the invention can be made by standard methods known in the art, including standard chemical synthesis and transcription of DNA encoding them. In addition, snoRNAs can be targeted to particular cells by coupling them to ligands specific for receptors on the cell surface of a target cell. snoRNAs can also be targeted to specific cell types by being conjugated to monoclonal antibodies that specifically bind to cell-type-specific receptors.

The nucleic acid constructs and/or vectors of the present invention may comprise more than one sequence designed to produce a modified snoRNA according to the present invention. When more than one sequence is present, the sequences may be designed to target the same target nucleic acid, or different target nucleic acids.

The nucleic acid constructs, vectors or indeed the nucleic acid encoding the modified snoRNA itself, may be designed to express further molecules such as RNAi molecules to be used in conjunction with the snoRNA molecules of the present invention, to modulate gene expression.

It may also be possible to generate disease models using the techniques of the present invention such that conventional transgenic animals do not need to be generated. Thus, the nucleic acid, nucleic acid construct or vectors of the present invention may be designed to express a snoRNA to prevent or minimise expression of a normal wild-type gene/protein in a host cell/animal and also to express a mutant form of the gene/protein associated with a particular disease/condition. Potential therapies may then be added/administered to the cell/animal in order to see if the therapy can treat or ameliorate the effect of the mutant gene/protein.

The snoRNAs, nucleic acids, nucleic acid constructs and/or vectors of the invention may be used for determining the function of a gene in a cell or an organism or even for modulating the function of a gene in a cell or an organism. The cell is preferably a eukaryotic cell or a cell line, e.g. a plant cell or an animal cell, such as a mammalian cell, e.g. an embryonic cell, a pluripotent stem cell, a tumor cell, e.g. a teratocarcinoma cell or a virus-infected cell. The organism is preferably a eukaryotic organism, e.g. a plant or an animal, such as a mammal, particularly a human.

The target gene to which the snoRNA molecules of the invention are directed may be associated with a pathological condition. For example, the gene may be a pathogen-associated gene, e.g. a viral gene, a tumor-associated gene or an autoimmune disease-associated gene. The target gene may also be a heterologous gene expressed in a recombinant cell or a genetically altered organism. By determinating or modulating, particularly, inhibiting the function of such a gene valuable information and therapeutic benefits in the agricultural field or in the medicine or veterinary medicine field may be obtained. The present invention allows for the provision of nucleic acid constructs and snoRNAs designed to replace one form of a cellular (or viral) protein (or functional RNA) with an alternative form, typically using a single vector. For example, this can be used to correct defects found in an organism or cell, such as a mutant gene that may be known to be the causative agent of a disease (e.g. gene mutation in the CFTR gene leading to the development of cystic fibrosis). The same vector can be designed to encode snoRNA(s) targeted to reduce expression of the mutant gene and also encode a correct copy of the gene, within the same construct, to express the correct protein. The correct copy can be provided by way of a cDNA sequence, or alternatively be encoded by the exons, which flank the introns encoding the modified snoRNAs of the present invention. Other examples include the replacement of mutant p53 genes, BRCA genes and the like associated with cancer progression.

Advantageously, the snoRNA and replacement nucleic acid may be located within the same transcript and expressed from the same promoter as the snoRNAs that knock down expression. This has the advantage that it allows everything to be expressed as a single transcript. Moreover, this also has the advantage that it makes the expression level of the snoRNAs and the expressed replacement nucleic acid balanced and co-regulated (ie from the same promoter).

A similar approach can be used to replace a targeted RNA that does not encode a protein (e.g. snRNA or miRNA or other functional RNA) with a modified form of the same RNA.

This strategy is not confined to correcting defects. A similar strategy can be used to replace any protein (or RNA), either encoded by a cell or virus, including a wild type and/or functional form of the protein (or RNA) being replaced with a defective, mutant or modified form of the same gene product, which may be desirable for certain applications, for example to assess mutant phenotypes, or to aid drug screening strategies, or for target validation, or to analyse a tagged form of a protein without competition from the existing, untagged cellular form, or for other applied or research applications. The strategy can be employed also to replace a protein (or RNA) of one type with a completely or substantially different protein (or RNA), using a single vector.

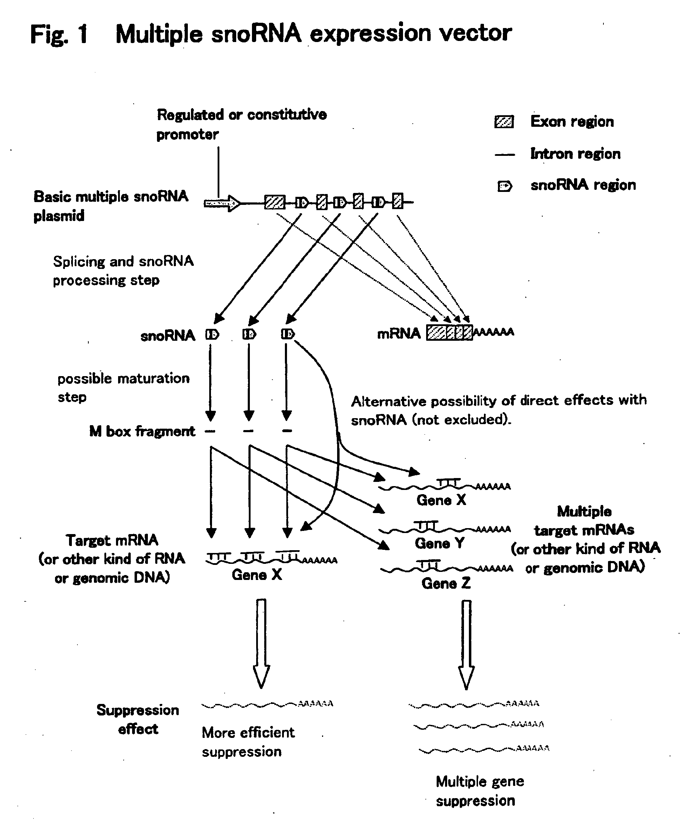

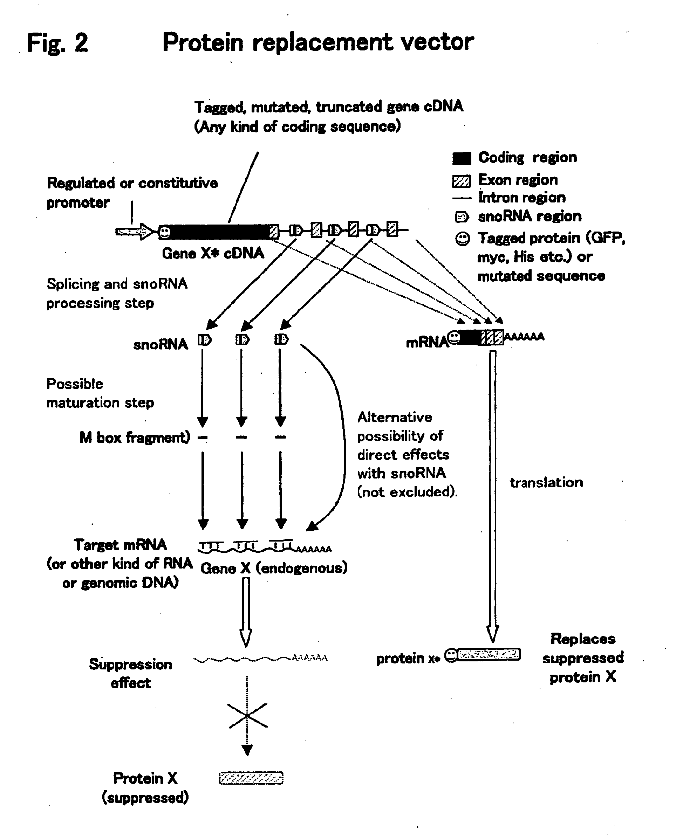

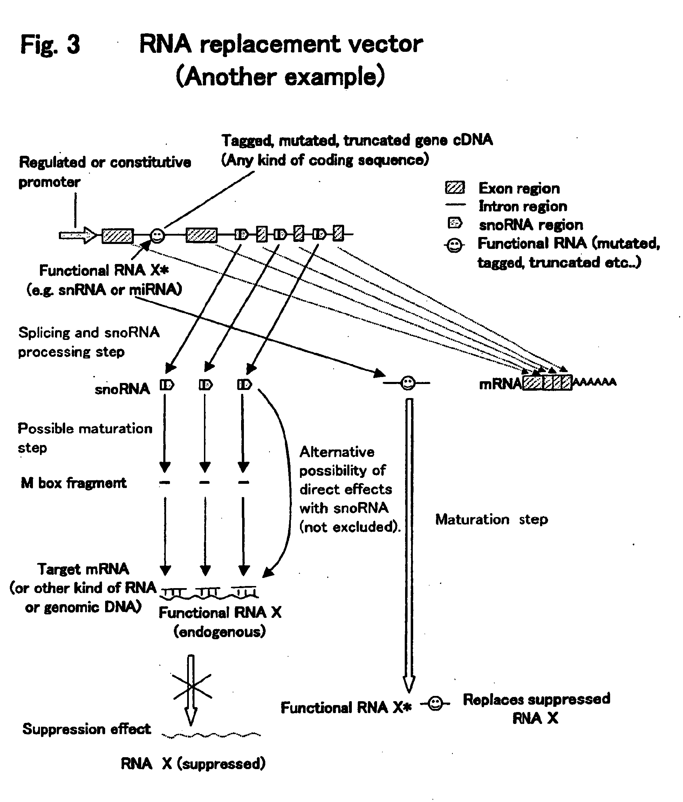

Figures 1-3 show schematically examples of vectors which may be used in accordance with the present invention. In figure 1, the vector shown as expressing multiple snoRNAs, which can be targeted to modulate expression a single gene sequence, or multiple gene sequences. Figure 2 shows schematically a protein replacement vector, which is designed to provide snoRNA(s) which inhibit expression of a target gene X, which can be replaced by a new protein encoded for by cDNA found within the vector. Figure 3 shows schematically a similar vector to the protein replacement vector, but which is for inhibiting an RNA, such as a mutant RNA and which can be replaced with a new/different version from sequence present in the vector.

Further applications for the cell or organism of the invention is the analysis of gene expression profiles and/or proteomes. In an embodiment an analysis of a variant or mutant form of one or several target proteins may be carried out, wherein said variant or mutant forms are reintroduced into the cell or organism by an exogenous target nucleic acid as described above. The combination of knockout of an endogenous gene and rescue by using mutated, e.g. partially deleted exogenous target has advantages compared to the use of a knockout cell. Further, this method is particularly suitable for identifying functional domains of the target protein. In a further preferred embodiment a comparison, e.g. a gene expression profiles and/or proteomes and/or phenotypic characteristics of at least two cells or organisms is carried out.

The invention may also find application in a procedure for identifying and/or characterising pharmacological agents, e.g. identifying new pharmacological agents from a collection of test substances and/or characterising mechanisms of action and/or side effects of known pharmacological agents.

In a further aspect, there is provided a snoRNA, nucleic acid, nucleic acid construct or vector according to the present invention for use in the manufacture of a medicament for use in treating a disease or condition in a subject where it is desirable to modulate, especially down-regulate expression of a target gene or genes.

As used herein the phrase "treating" refers to inhibiting or arresting the development of a disease, disorder or condition and/or causing the reduction, remission, or regression of a disease, disorder or condition in an individual suffering from, or diagnosed with, the disease, disorder or condition. Those of skill in the art will be aware of various methodologies and assays which can be used to assess the development of a disease, disorder or condition, and similarly, various methodologies and assays which can be used to assess the reduction, remission or regression of a disease, disorder or condition.

The method according to this aspect of the present invention is effected by providing to cells of the individual the isolated polynucleotide of the present invention to thereby downregulate the level of a target RNA in the cells of the individual.

Providing can be effected by directly administering the polynucleotide of the present invention into the cells or by expressing the polynucleotide in cells as described hereinabove. Expressing can be effected by directly transfecting cells of the individual with a nucleic acid construct capable of expressing the polynucleotide of the present invention (i.e., *in vivo* transfection), or by transfecting cells isolated from the individual with the nucleic acid construct and administering the transfected cells to the individual (i.e., *ex vivo* transfection).

In a further aspect there is provided a pharmaceutical composition comprising a snoRNA according to the present invention, or a nucleic acid construct capable of expressing a snoRNA molecule according to the present invention and a pharmaceutically acceptable carrier therefore.

Pharmaceutical formulations include those suitable for oral, topical (including dermal, buccal and sublingual), rectal or parenteral (including subcutaneous, intradermal, intramuscular and intravenous), nasal and pulmonary administration e.g., by inhalation. The formulation may, where appropriate, be conveniently presented in discrete dosage units and may be prepared by any of the methods well known in the art of pharmacy. All methods include the step of bringing into association an active compound with liquid carriers or finely divided solid carriers or both and then, if necessary, shaping the product into the desired formulation.

Pharmaceutical formulations suitable for oral administration wherein the carrier is a solid are most preferably presented as unit dose formulations such as boluses, capsules or tablets each containing a predetermined amount of active compound. A tablet may be made by compression or moulding, optionally with one or more accessory ingredients. Compressed tablets may be prepared by compressing in a suitable machine an active compound in a free-flowing form such as a powder or granules optionally mixed with a binder, lubricant, inert diluent, lubricating agent, surface-active agent or dispersing agent. Moulded tablets may be made by moulding an active compound with an inert liquid diluent. Tablets may be optionally coated and, if uncoated, may optionally be scored. Capsules may be prepared by filling an active compound, either alone or in admixture with one or more accessory ingredients, into the capsule shells and then sealing them in the usual manner. Cachets are analogous to capsules wherein an active compound together with any accessory ingredient(s) is sealed in a rice paper envelope. An active compound may also be formulated as dispersable granules, which may for example be suspended in water before administration, or sprinkled on food. The granules may be packaged, e.g., in a sachet. Formulations suitable for oral administration wherein the carrier is a liquid may be presented as a solution or a suspension in an aqueous or non-aqueous liquid, or as an oil-in-water liquid emulsion.

Formulations for oral administration include controlled release dosage forms, e.g., tablets wherein an active compound is formulated in an appropriate release - controlling matrix, or is coated with a suitable release - controlling film. Such formulations may be particularly convenient for prophylactic use.

Pharmaceutical formulations suitable for rectal administration wherein the carrier is a solid are most preferably presented as unit dose suppositories. Suitable carriers include cocoa butter and other materials commonly used in the art. The suppositories may be conveniently formed by admixture of an active compound with the softened or melted carrier(s) followed by chilling and shaping in moulds.

Pharmaceutical formulations suitable for parenteral administration include sterile solutions or suspensions of an active compound in aqueous or oleaginous vehicles.

Injectible preparations may be adapted for bolus injection or continuous infusion. Such preparations are conveniently presented in unit dose or multi-dose containers which are sealed after introduction of the formulation until required for use. Alternatively, an active compound may be in powder form which is constituted with a suitable vehicle, such as sterile, pyrogen-free water, before use.

An active compound may also be formulated as long-acting depot preparations, which may be administered by intramuscular injection or by implantation, e.g., subcutaneously or intramuscularly. Depot preparations may include, for example, suitable polymeric or hydrophobic materials, or ion-exchange resins. Such long-acting formulations are particularly convenient for prophylactic use.

Formulations suitable for pulmonary administration via the buccal cavity are presented such that particles containing an active compound and desirably having a diameter in the range of 0.5 to 7 microns are delivered in the bronchial tree of the recipient.

As one possibility such formulations are in the form of finely comminuted powders which may conveniently be presented either in a pierceable capsule, suitably of, for example, gelatin, for use in an inhalation device, or alternatively as a self-propelling formulation comprising an active compound, a suitable liquid or gaseous propellant and optionally other ingredients such as a surfactant and/or a solid diluent. Suitable liquid propellants include propane and the chlorofluorocarbons, and suitable gaseous propellants include carbon dioxide. Self-propelling formulations may also be employed wherein an active compound is dispensed in the form of droplets of solution or suspension.

Such self-propelling formulations are analogous to those known in the art and may be prepared by established procedures. Suitably they are presented in a container provided with either a manually-operable or automatically functioning valve having the desired spray characteristics; advantageously the valve is of a metered type delivering a fixed volume, for example, 25 to 100 microlitres, upon each operation thereof.

As a further possibility an active compound may be in the form of a solution or suspension for use in an atomizer or nebuliser whereby an accelerated airstream or ultrasonic agitation is employed to produce a fine droplet mist for inhalation.

Formulations suitable for nasal administration include preparations generally similar to those described above for pulmonary administration. When dispensed such formulations should desirably have a particle diameter in the range 10 to 200 microns to enable retention in the nasal cavity; this may be achieved by, as appropriate, use of a powder of a suitable particle size or choice of an appropriate valve. Other suitable formulations include coarse powders having a particle diameter in the range 20 to 500 microns, for administration by rapid inhalation through the nasal passage from a container held close up to the nose, and nasal drops comprising 0.2 to 5% w/v of an active compound in aqueous or oily solution or suspension.

It should be understood that in addition to the aforementioned carrier ingredients the pharmaceutical formulations described above may include, an appropriate one or more additional carrier ingredients such as diluents, buffers, flavouring agents, binders, surface active agents, thickeners, lubricants, preservatives (including anti-oxidants) and the like, and substances included for the purpose of rendering the formulation isotonic with the blood of the intended recipient.

Pharmaceutically acceptable carriers are well known to those skilled in the art and include, but are not limited to, 0.1 M and preferably 0.05 M phosphate buffer or 0.8% saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Preservatives and other additives may also be present, such as, for example, antimicrobials, antioxidants, chelating agents, inert gases and the like.

Formulations suitable for topical formulation may be provided for example as gels, creams or ointments. Such preparations may be applied e.g. to a wound or ulcer either directly spread upon the surface of the wound or ulcer or carried on a suitable support such as a bandage, gauze, mesh or the like which may be applied to and over the area to be treated.

Liquid or powder formulations may also be provided which can be sprayed or sprinkled directly onto the site to be treated, e.g. a wound or ulcer. Alternatively, a carrier such as a bandage, gauze, mesh or the like can be sprayed or sprinkle with the formulation and then applied to the site to be treated.

Therapeutic formulations for veterinary use may conveniently be in either powder or liquid concentrate form. In accordance with standard veterinary formulation practice, conventional water soluble excipients, such as lactose or sucrose, may be incorporated in the powders to improve their physical properties. Thus particularly suitable powders of this invention comprise 50 to 100% w/w and preferably 60 to 80% w/w of the active ingredient(s) and 0 to 50% w/w and preferably 20 to 40% w/w of conventional veterinary excipients. These powders may either be added to animal feedstuffs, for example by way of an intermediate premix, or diluted in animal drinking water.

Liquid concentrates of this invention suitably contain the compound or a derivative or salt thereof and may optionally include a veterinarily acceptable water-miscible solvent, for example polyethylene glycol, propylene glycol, glycerol, glycerol formal or such a solvent mixed with up to 30% v/v of ethanol. The liquid concentrates may be administered to the drinking water of animals.

### Detailed Description

The present invention will now be described with reference to the following non-limiting examples and figures, which show:
**Figure 1****:** a schematic representation of a snoRNA expression vector according to the present invention designed to express multiple snoRNAs;
**Figure 2****:** a schematic representation of a snoRNA expression vector designed to replace a host cell protein with a vector encoded protein:
**Figure 3****:** a schematic representation of a snoRNA expression vector designed to replace a host cell RNA with a vector encoded RNA.
**Figure 4****: HeLa cell Nucleolar RNA library.**
   (A) Isolation and cloning of Nucleolar RNA from HeLa cell nucleolar fraction. The left panel shows intact HeLa cell image before fractionation and right panel shows isolated nucleoli. HeLa cell nucleoli were fractionated by the sucrose gradient method as previously described (Andersen et. al., 2005), then total nucleolar RNA was isolated from the nucleolar pellet. Total nucleolar RNA was modified by addition of poly adenosine at the 3' end using poly A polymerase.
   (B) Schematic diagram showing cDNA synthesis strategy which was performed both with amplification (shown on right) and without amplification (shown on left). Nucleolar cDNAs were synthesized by reverse transcription using PolyA tailed nucleolar RNA as the template. The library made without amplification (left) was mainly used to detect abundant nucleolar RNA species, while we used the amplification technique (right) to make a library including lower abundance nucleolar RNA species. Both of these libraries were independently cloned into the general plasmid vectors and analyzed by DNA sequencing.
   (C) Summary of RNA species detected in the nucleolar cDNA libraries. We analyzed the amplified and non-amplified libraries independently but the pie chart shows the combined result for both libraries.

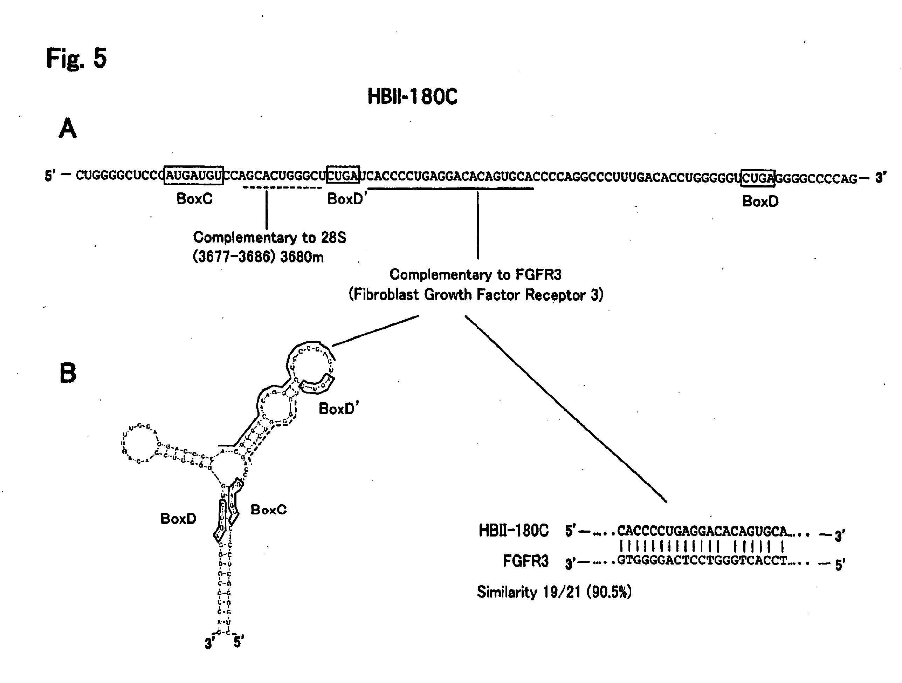

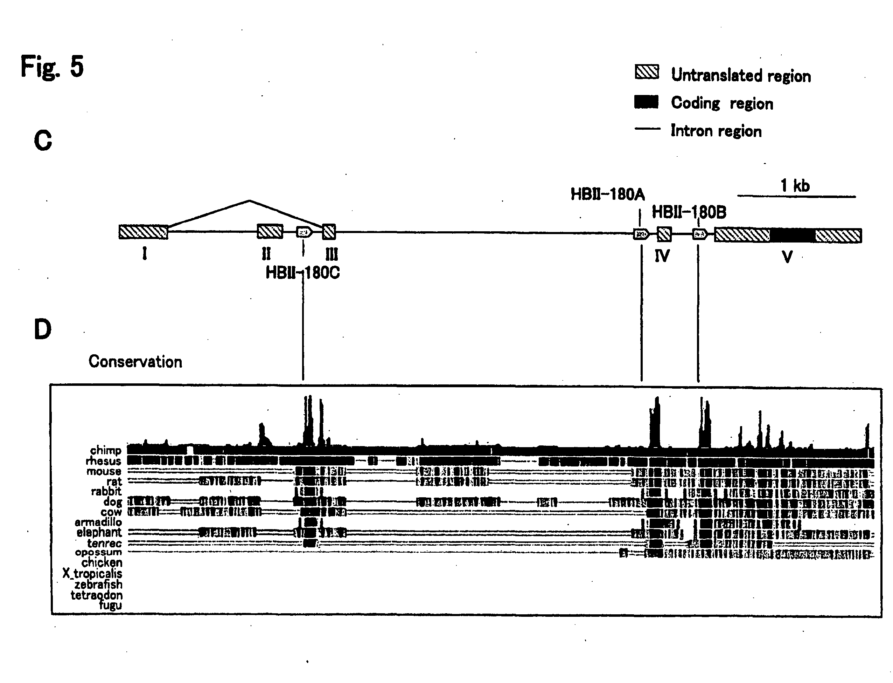

**Figure 5****: Conservation of HBII-180C snoRNA and host gene C19orf48.**
   (A) Structure of the HBII-180C snoRNA. BoxC (A/G)UGAUGA) and boxes D or D' (CUGA) are each indicated within boxes. The predicted rRNA complementary sequence for specifying the ribose 2'-O- methylation site is indicated by an underline.
   (B) Predicted secondary structure of HBII-180C snoRNA indicating the region found to be complementary to FGFR3 pre-mRNA. Secondary structure of HBII-180C snoRNA was calculated by mfold (http://frontend.bioinfo.rpi.edu/applications/mfold/cgi-bin/rna-form1.cgi) (M. Zuker Mfold web server for nucleic acid folding and hybridization prediction. Nucleic Acids Res. 31 (13), 3406-15, (2003) and D.H. Mathews, J. Sabina, M. Zuker and D.H. Turner Expanded Sequence Dependence of Thermodynamic Parameters Improves Prediction of RNA Secondary Structure J. Mol. Biol. 288, 911-940 (1999) (left). BoxC and boxes D and D' are each indicated within boxes. rRNA complementary sequence (dotted line) and FGFR3 complementary sequence (solid line) are indicated with bars. The complementary region of FGFR3 pre-mRNA is indicated on the right, showing the potential base pairing interaction.
   (C) Structure of the human C19orf48 gene. Exons are indicated as striped boxes with roman numbers. Predicted protein coding region is indicated in black. The intron-encoded snoRNAs HBII-180A, HBII-180B and HBII-180C are indicated as solid grey boxes.
   (D) Conserved region of C19orf48 gene. This picture is derived from C19orf48 chromosome position of UCSC Human Genome Browser (http://genome.ucsc.edu/cgi-bin/hgGateway Kent WJ, Sugnet CW, Furey TS, Roskin KM, Pringle TH, Zahler AM, Haussler D: The human genome browser at UCSC. Genome Res 2002, 12(6):996-1006) Vertebrate Multiz Alignment and conservation results are indicated as black boxes and bar. Conserved regions among 17 vertebrate species are indicated as bars (Conservation). The three most conserved regions correspond exactly to the three detected HBII snoRNAs.

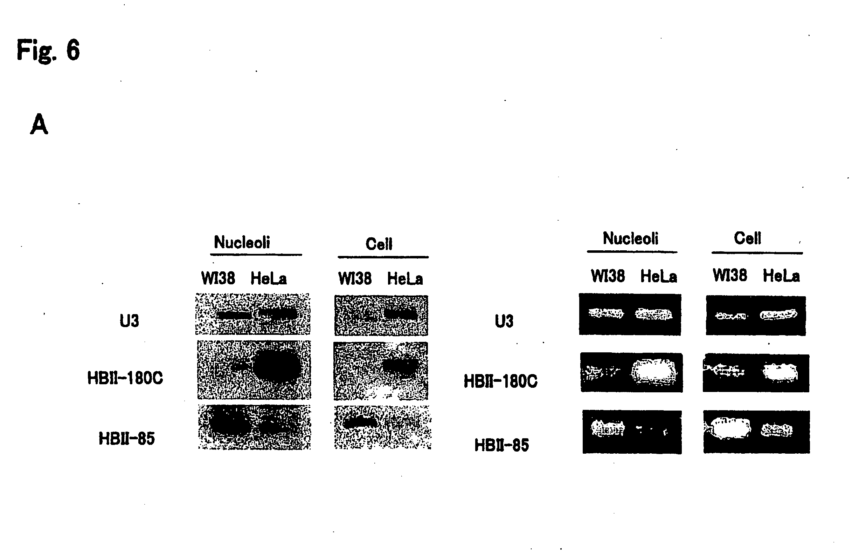

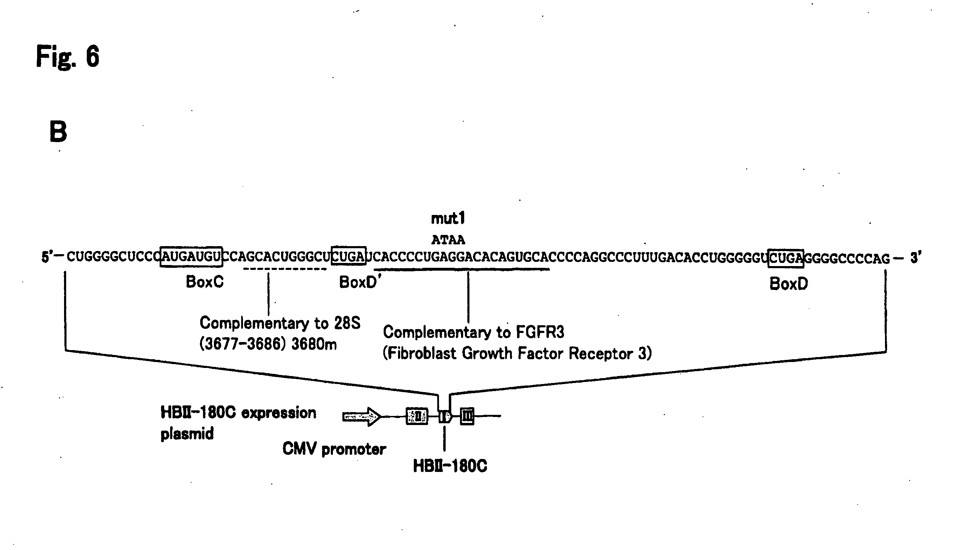

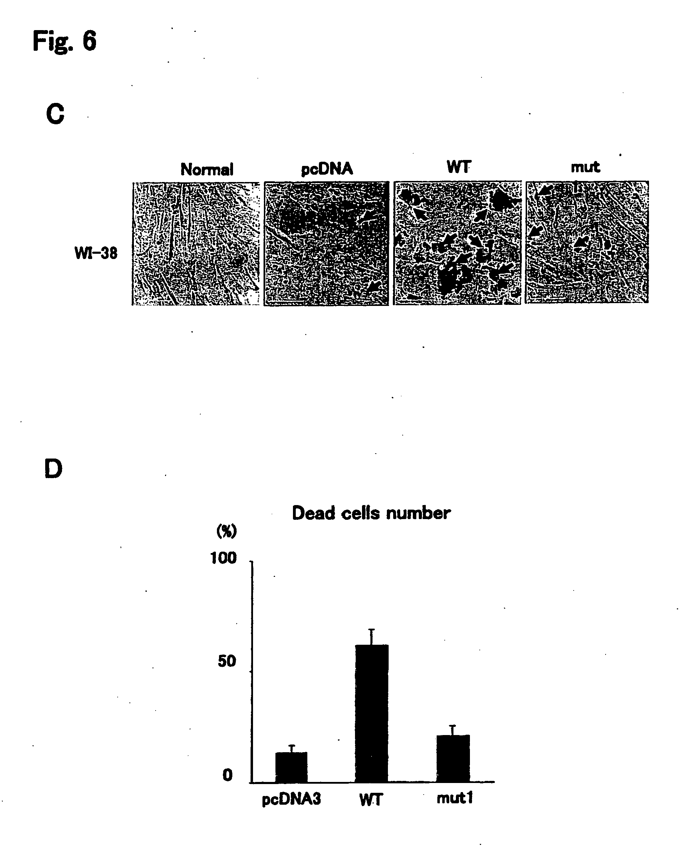

**Figure 6****: The sequence specific effect of FGFR3 complementary region of HBII-180C.**
   (A) HBII-180C expression levels analysed both by northern blot (left panels) and by semi-quantitative RT-PCR (right panels). Left panels show the results of northern blot analysis using a HBII-180C specific probe. The same amount of either total nucleolar RNA (Nucleoli), or total cell RNA (Cell) from human primary cell line WI38 (WI38) and HeLa cells (HeLa) were blotted. Right panels show the results of semi-quantitative RT-PCR using a HBII-180C specific primer set. The same amount of either total nucleolar RNA, or total cell RNA from WI38 and HeLa cells, were used as template.
   (B) The HBII-180C expression plasmid for wild type and mutant HBII-180C snoRNAs. The sequence in HBTI-180C that is complementary to FGFR3 pre-mRNA was changed from GAGG to ATAA (blue, mut1).
   (C) Comparison of the effect of expressing either wt or mut1 HBII-180C snoRNAs in WI38 cells. Images show representative examples of cells three days after transfection of WI38 cells with either empty plasmid pcDNA3 (pcDNA3), HBII-180C wild type expression plasmid (WT) or HBII-180C mutant expression plasmid (mut1). Red arrows indicate clumps of either dying cells or dead cells.
   (D) Graph showing percentage of dead cells three days after expression with the plasmids described above. The mutation of the FGFR3 pre-mRNA complementary region clearly decreased the level of cell death as compared with WT.

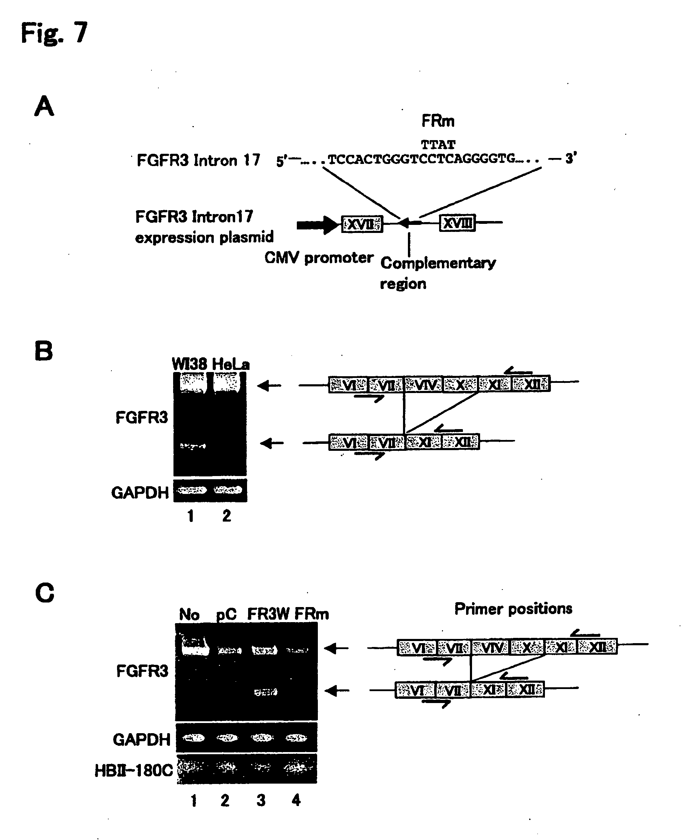

**Figure 7****: The FGFR3-complementary region of HBD-180C snoRNA is associated with the pattern of alternative FGFR3 spliced isoforms.**
   (A) The FGFR3 intron 17 expression plasmid for wild type and mutant, designed to suppress expression/activity of endogenous HBII180-C snoRNA. For the mutant construct the complementary region of FGFR3 was changed from CCTC to TTAT (blue, FRm).
   (B) The endogenous expression patterns for FGFR3 spliced mRNA isoforms in both WI38 and HeLa cells. Gel (left image) shows the results of RT-PCR analysis to detect alternative FGFR3 spliced mRNA isoforms. Diagram on right shows the positions for the FGFR3 mRNA specific primer set. Exons are indicated as boxes with roman numbers. Primers used for PCR are shown as arrows.
   (C) FGFR3 alternatively spliced RNA isoform pattern was changed by over expression of the FGFR3 mini gene. Gel image shows the results of RT-PCR without transfection (No, lane 1), transfected empty plasmid pcDNA3.1 (pC, lane 2), wild type mini gene of FGFR3 intron 17 (FR3W, lane 3) and mutant mini gene (FRm, lane 4).

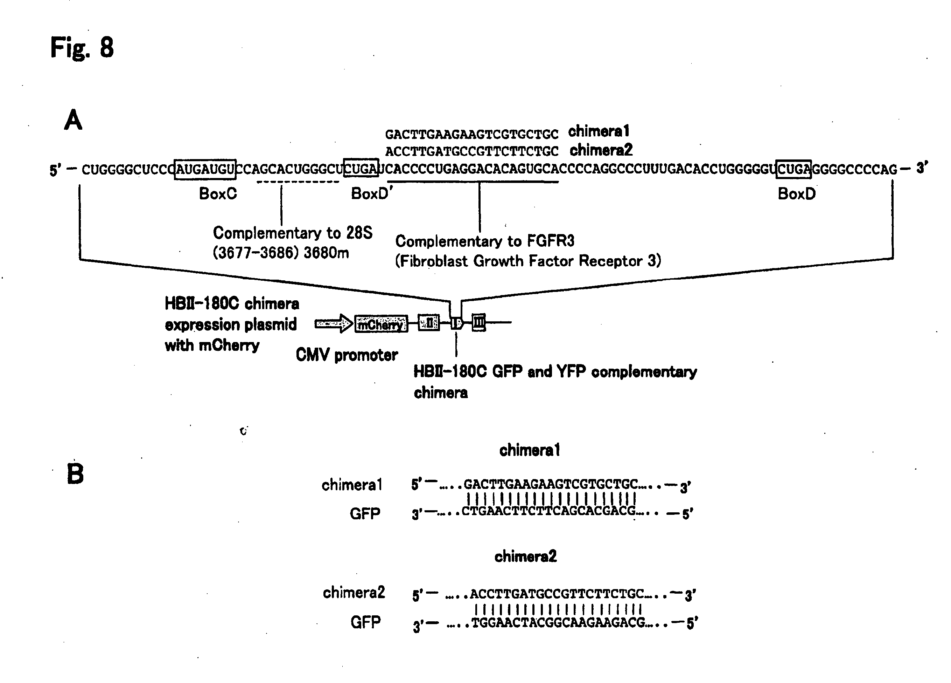

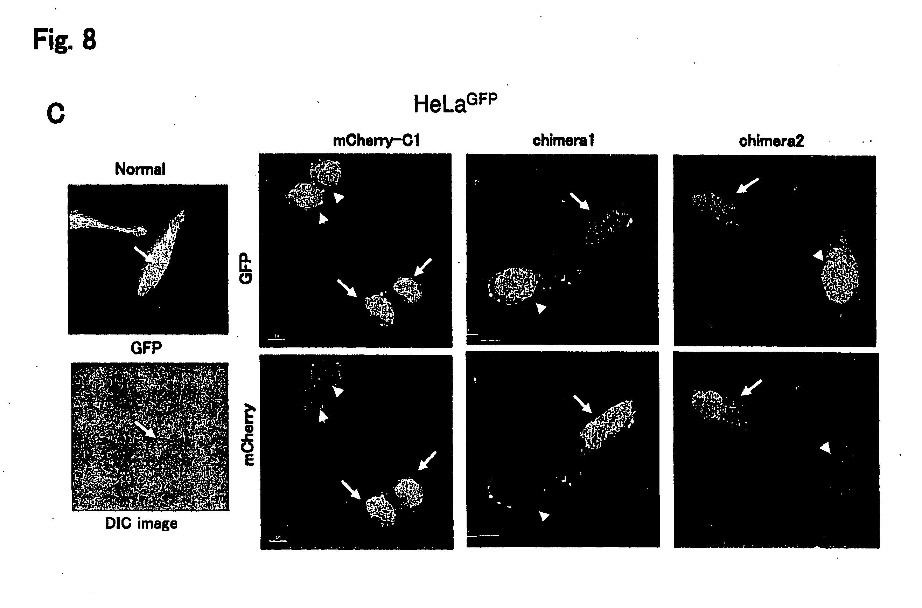

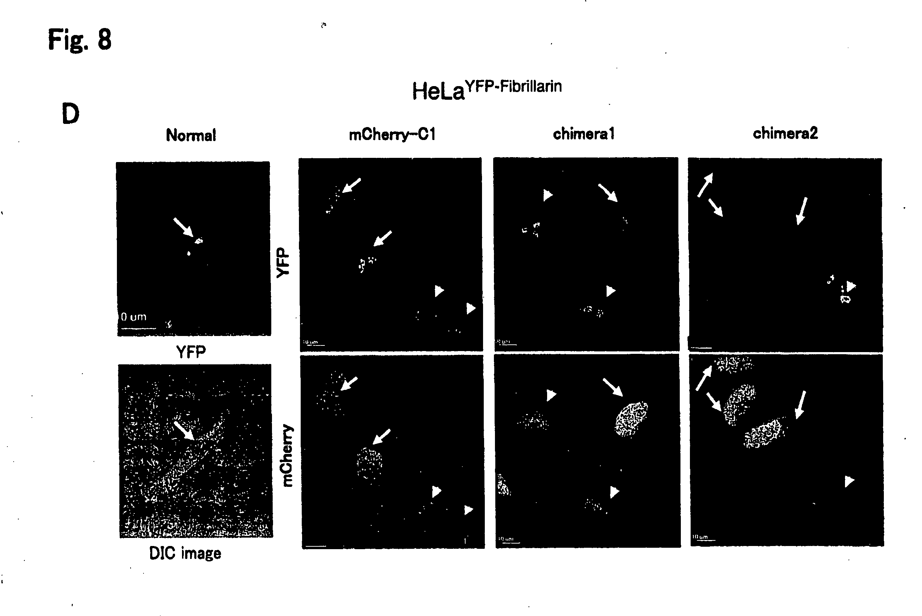

**Figure 8****: Targeted suppression of GFP and YFP using a modified snoRNA expression vector.**
   (A) Chimera expression plasmid targeting sequences shared by GFP and YFP. The sequence in WT HBII-180C snoRNA that is complementary to FGFR3 pre-mRNA was changed from 5'-CACCCCAGAGGACACAGTGCA-3' to either 5'-GACTTGAAGAAGTCGTGCTGC-3' (blue, chimeral) or to 5'-ACCTTGATGCCGTTCTTCTGC-3' (blue, chimera2). The resulting Chimera 1&2 snoRNAs were subcloned into the 3' region of the vector expressing mCherry fluorescent protein cDNA.
   (B) Diagram shows complementary regions of chimera1 and chimera2 targeting two separate regions of GFP, both of which are also present in the related YFP gene.
   (C) The effect of chimera constructs on GFP expression in the HeLa^{GFP} stable cell line, which expresses unfused GFP alone. Images show the effect of transfecting either empty mCherry expression plasmid mCherry-C1 (mCherry-C1), expression plasmid HBII-180C chimera1 or expression plasmid HBII-180C chimera2 in the HeLa^{GFP} stable cell line. The left panel shows a live cell image of the HeLa^{GFP} stable cell line without transfection (DIC image). Upper panels show GFP fluorescence signals of images recorded from fixed cells (Green). Lower panels show merged images combining the GFP (green) and mCherry (red) signals. The arrows indicate transfected cells and arrowheads show untrasfected cells. Note clear reduction in GFP expression specifically in the cells transfected with the expression plasmids encoding either HBII-180C chimera1 or 2.
   (D) The effect of chimera constructs on YFP expression in the HeLa^{YFP-FIBRILLARIN} stable cell line, which expresses YFP fused at the amino terminus of fibrillarin (Leung et. al., 2004). Pictures show the effect of transfections using either empty mCherry expression plasmid mCheny-C1 (mCherry-C1), expression plasmid HBII-180C chimera1 or expression plasmid HBII-180C chimera2 in the HeLa^{YFP-FIBRILLARIN} stable cell line. The left panel shows a live cell image of the HeLa^{YFP-FIBRILLARIN} stable cell line without transfection (DIC image). Upper panels show YFP fluorescence signals of images recorded from fixed cells (Green). Lower panels are merged images combining the YFP (Green), cherry (Red) and DAPI signals (Blue). The arrows indicate transfected cells while arrowheads show untransfected cells. Note clear reduction in YFP-fibrillarin expression specifically in the cells transfected with the expression plasmids encoding either HBII-180C chimera1 or 2. In some cells the YFP signal is almost completely eliminated, indicating efficient suppression of YFP-fibrillarin by the chimeric snoRNA constructs.

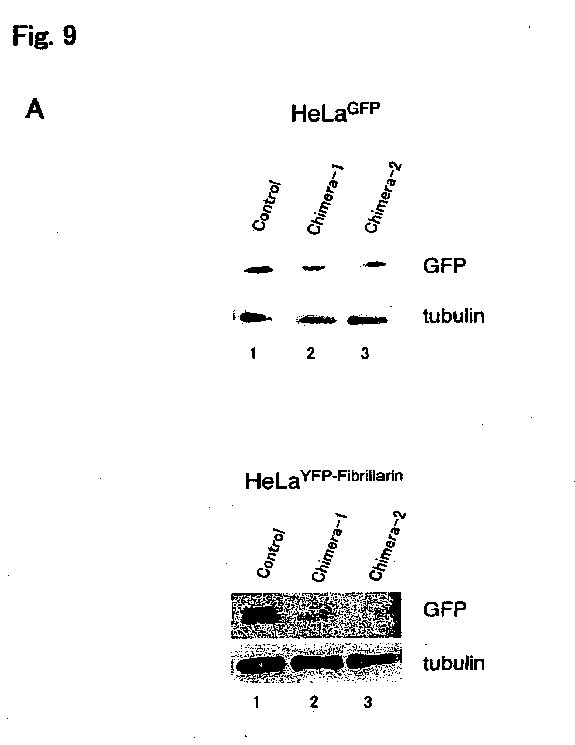

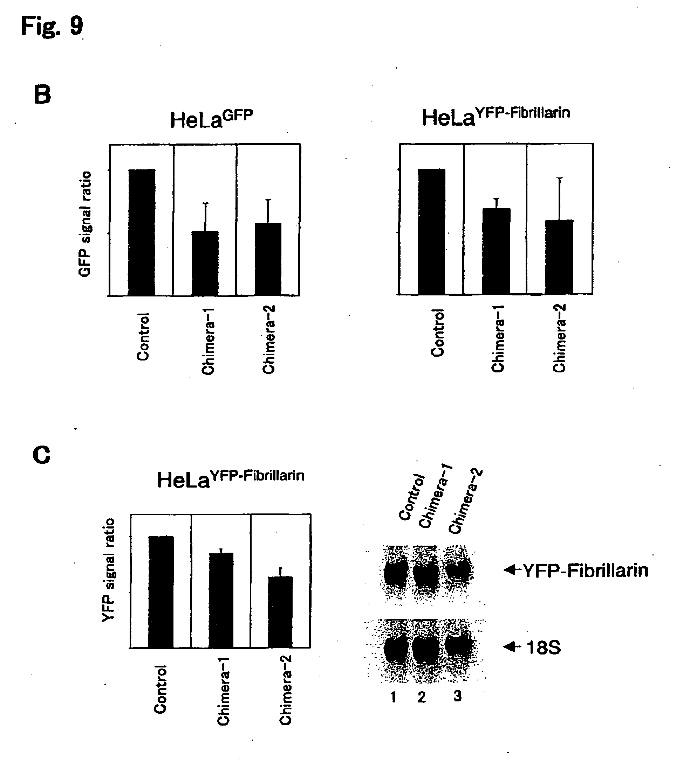

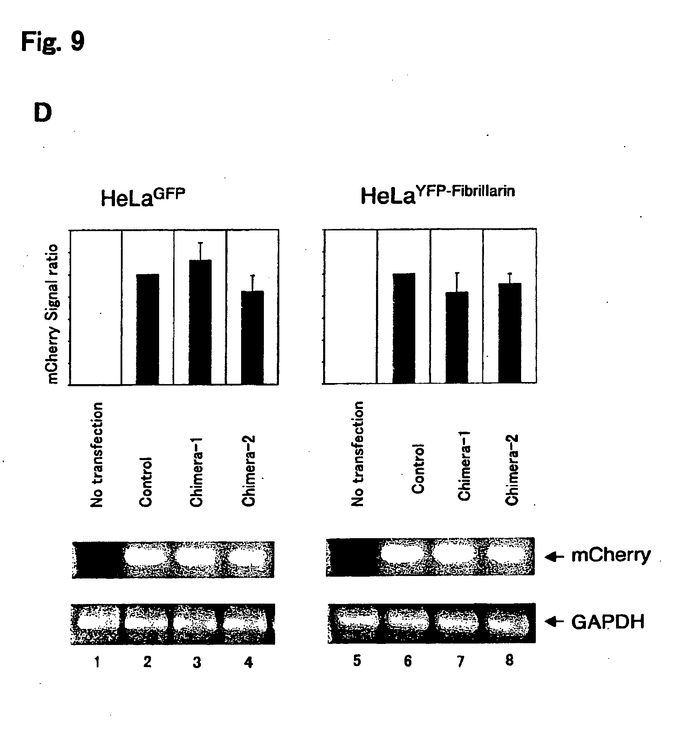

**Figure 9****: Targeted snoRNA suppression of GFP and YFP detected by Western and Northern blotting.**
   (A) Detection of protein levels for GFP (top) and YFP-Fibrillarin (bottom) following transfection of HeLa^{GFP} and HeLa^{YFP-FIBRILLARIN} stable cell lines using either empty mCherry expression plasmid mCherry-C1 (control: lane1), expression plasmid HBII-180C chimera1 (lane2) or expression plasmid HBII-180C chimera2 (lane3). An equivalent amount of HeLa extract was loaded for each lane and the proteins separated by SDS PAGE, electroblotted and probed both with a monoclonal anti-GFP antibody and with anti-tubulin as a loading control. Note reduction in GFP and YFP-fibrillarin levels, but not tubulin, specifically with the expression plasmids encoding either HBII-180C chimera1 or 2.
   (B) The graphs show average signal intensity and standard deviation for three independent experiments. GFP signal ratio was normalized to the tublin signal. Note reduction in GFP and YFP-fibrillarin levels, but not tubulin, specifically with the expression plasmids encoding either HBII-180C chimera1 or 2.
   (C) Detection of RNA levels of YFP-Fibrillarin following transfection of HeLa^{YFPFIBRILLARIN} stable cell lines using either empty mCherry expression plasmid mCherry-C1 (control: lane1), expression plasmid HBII-180C chimera1 (lane2) or expression plasmid HBII-180C chimera2 (lane3). An equivalent amount of HeLa cell total RNA was loaded for each lane and the RNA separated by 6.5% formaldehyde - 0.8% agarose gel, electroblotted and probed both with radio labeled anti-YFP oligo and with anti-18S rRNA as a loading control. The graphs show average signal intensity and standard deviation for three independent experiments. YFP-Fibrillarin signal ratio was normalized to the 18S rRNA signal.
   (D) Transfection efficiency for chimera snoRNA plasmids. mcherry mRNA was quantitated by RT-PCR. HeLa cell total RNA was isolated from transiently transfected mCherry plasmid (control) and chimera snoRNAs. The graphs show average signal intensity for three independent experiments. mCherry signal ratio was normalized to the GAPDH signal.

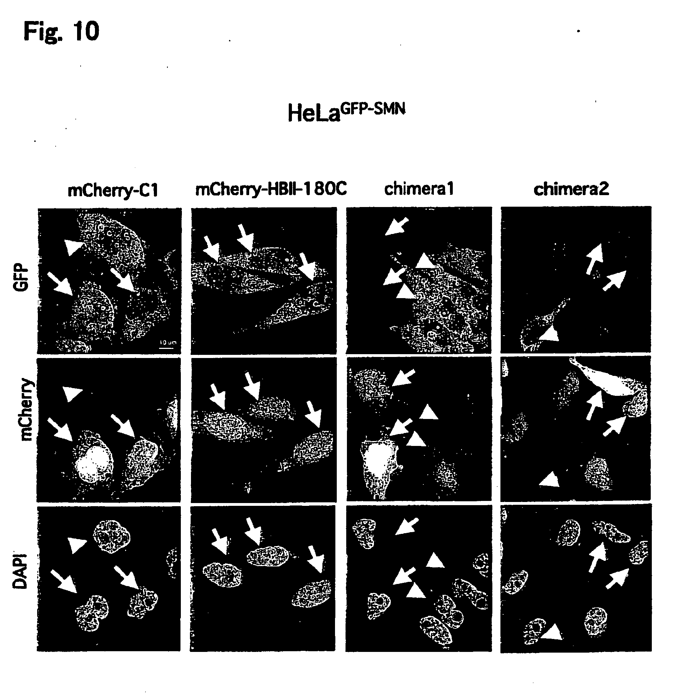

**Figure 10****: Another example of gene knock down using chimera1 & 2.**
   This is the same experimental design as shown in Figure 8, C &D but here using the stable cell line HeLa ^{GFP-SMN}. mCherry-HBII-180C is another negative control which has wild type HBII-180C snoRNA sequence in the 3' region of cherry cDNA. The arrows indicate transfected cells and arrowheads show untransfected cells. Note clear reduction in GFP expression specifically in the cells transfected with the expression plasmids encoding either HBII-180C chimera1 or 2.

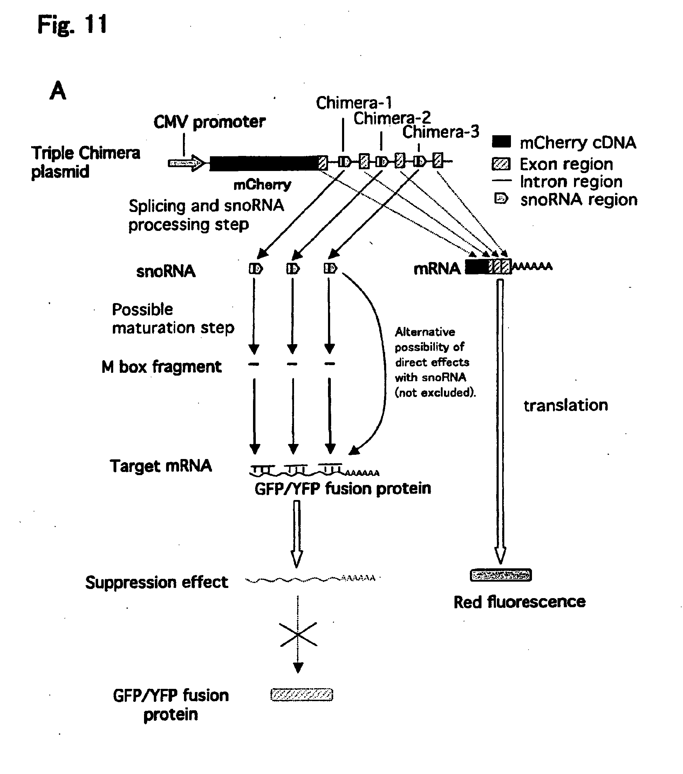

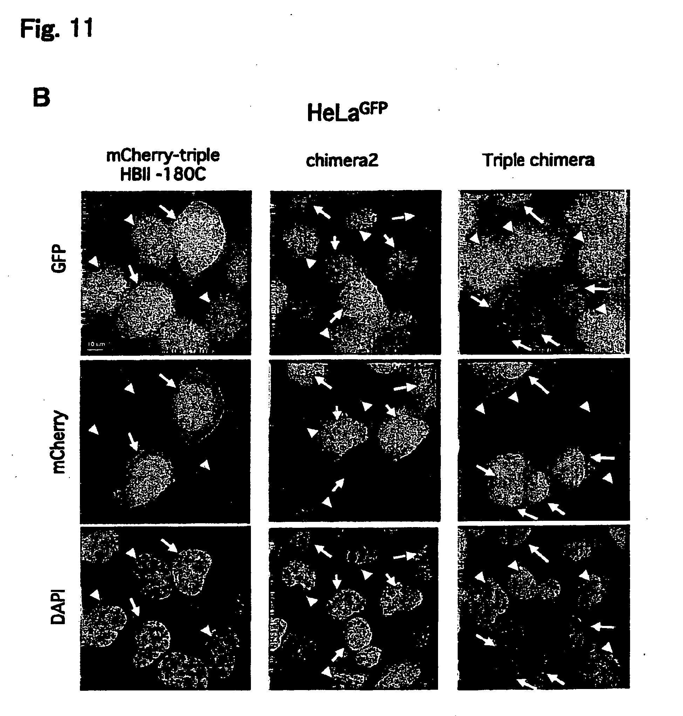

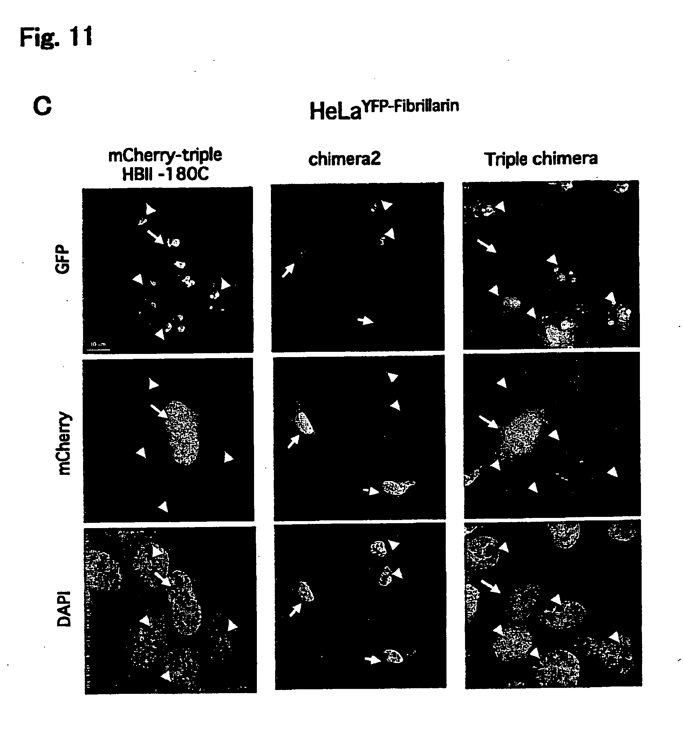

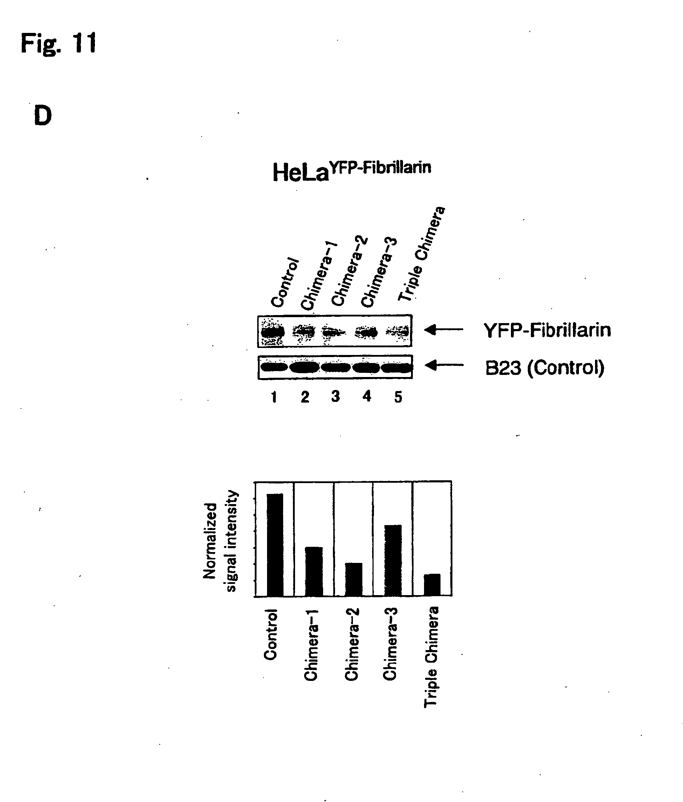

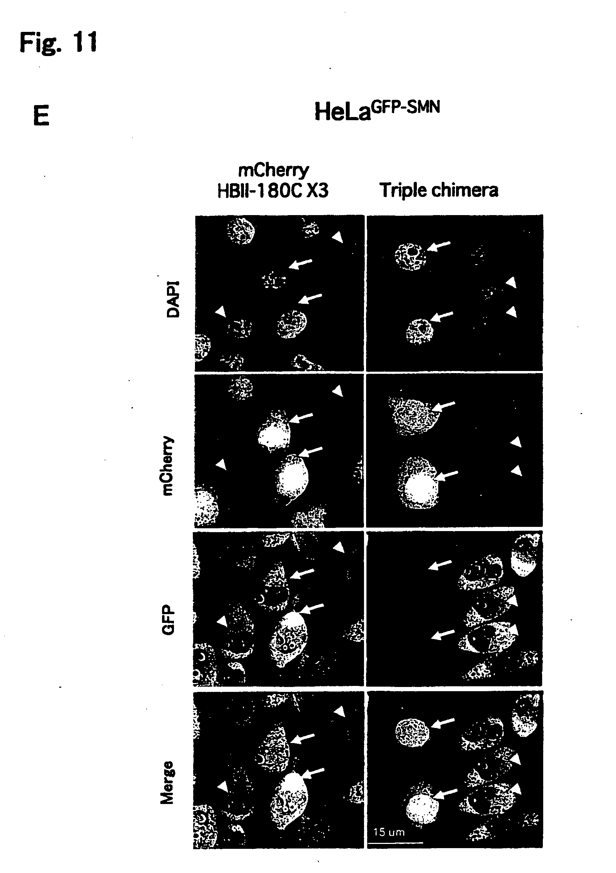

**Figure 11****: Targeted gene knockdown using triplet chimera snoRNA plasmid.**
   (A) Structure of triplet chimera snoRNA construct and schematic diagram of targeted GFP/YFP knock down.
   (B)(C) This is the same experimental design as used in Fig.8C & D but here using the chimera triplet plasmid for transfection. Mcherry-triple-HBII-180C is another negative control which has 3 repeats of wild type HBII-180C snoRNA sequence in 3' region of mCherry cDNA. Arrow: transfected, Arrowhead: untransfected.
   (D) Knock down efficiency of each chimera plasmid targeted to GFP/YFP was tested by western blotting (lane 1-5). Detection of protein levels for YFP-Fibrillarin following transfection of HeLa^{YFP-FIBRILLARIN} stable cell lines using either mCherry and wild type HBII-180C expression plasmid mCherry-HBII-180C (control: lane1), expression plasmid chimera1 (lane2), chimera2 (lane3), chimera3 (lane4), HBII-180C triple chimera (lane5). An equivalent amount of HeLa extract was loaded for each lane and the proteins separated by SDS PAGE, electroblotted and probed both with a monoclonal anti-GFP antibody and with anti-B23 as a loading control. Graph showed YFP-Fibrillarin signal intensity that have normalised by B23 signal. Note reduction in YFP-fibrillarin levels, but not B23, specifically with the expression plasmids encoding chimera1, 2 and 3. The efficiency of reducing the YFP-Fibrillarin expression level was enhanced by using the triplet chimera plasmid.
   (E) This is the same experimental design as shown in Fig. 11B & C but here using the chimera stable cell line HeLa ^{GFP-SMN}. The reduction of targeted gene expression mediated by the triple chimera plasmid was also detected using another stable cell line of GFP-SMN fusion protein. Arrow: transfected, Arrowhead: untransfected.

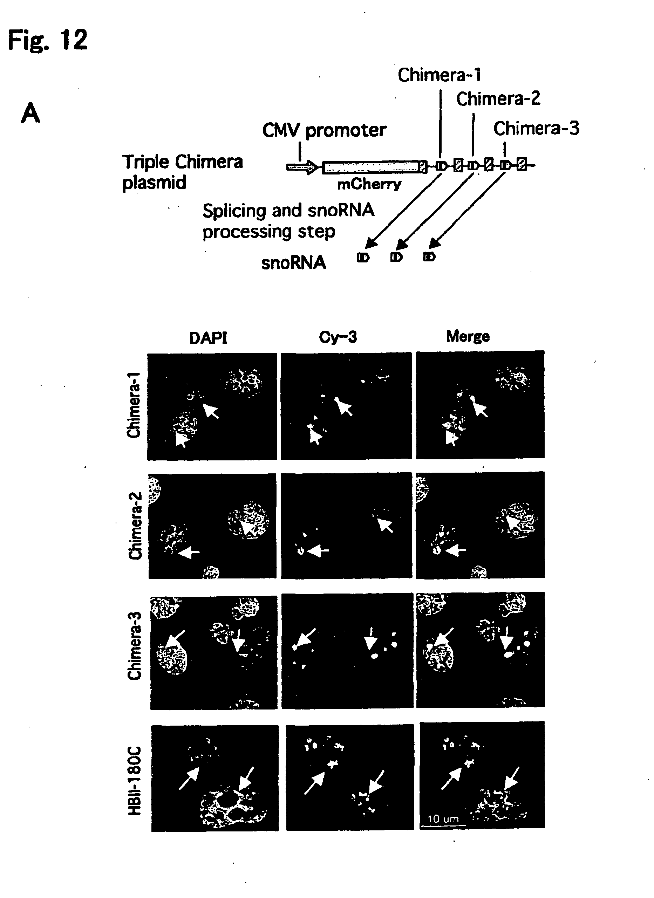

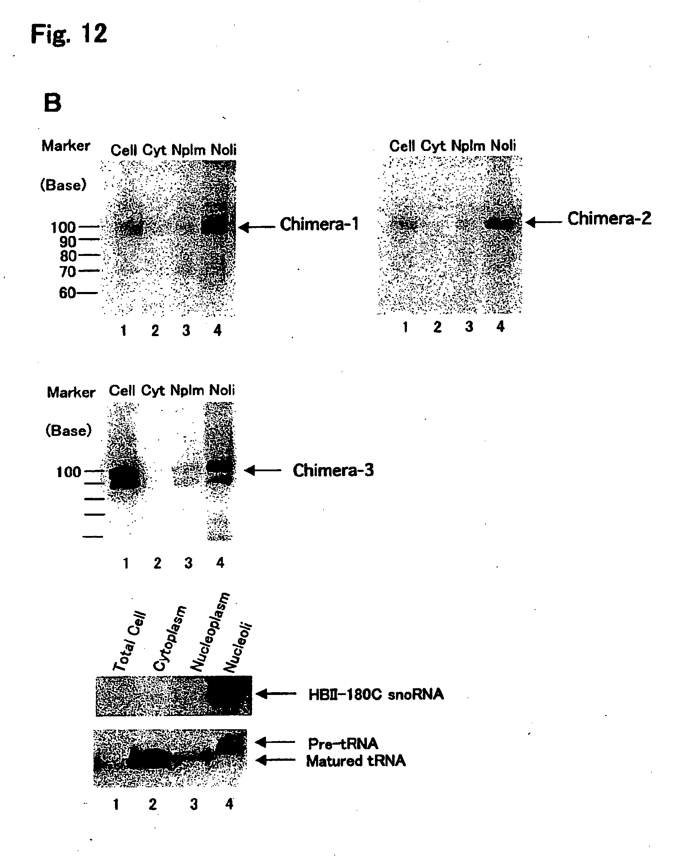

**Figure12****: Identification of chimera snoRNAs from chimera triplet plasmid.**
   (A) Fluorescence In Situ Hybridisation showing nucleolar localization of wild type HBII-180C and chimera snoRNAs (Cy3). DNA is stained by DAPI. Bar is 10 uM. Structure of triplet chimera snoRNA construct showed on top panel. Arrow shows nucleolus.
   (B) Characterization of HBII-180C and chimera snoRNAs. RNA blot showing sub cellular distribution of HBII-180C and Chimera snoRNAs. The same amount of HeLa cell total RNA (lanes 1), cytoplasmic RNA (lanes 2), nucleoplasmic RNA (lanes 3) and nucleolar RNA (lanes 4) were compared with either transiently transfected wild type or chimera snoRNA expression mini-genes by northern RNA blotting analysis using specific probes. Fractionation quality was tested using a tRNA-IIe specific probe (lower panel).

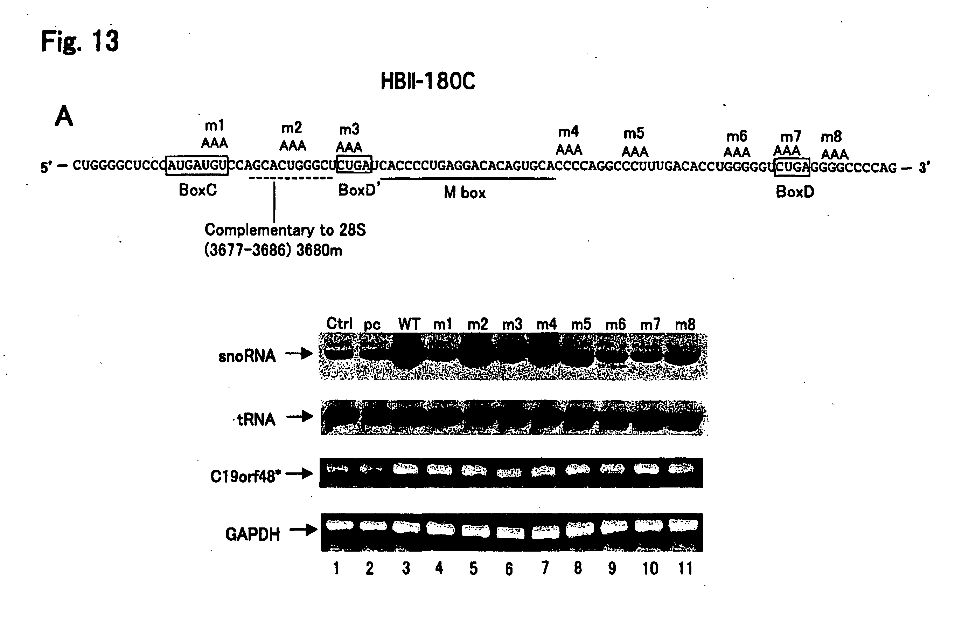

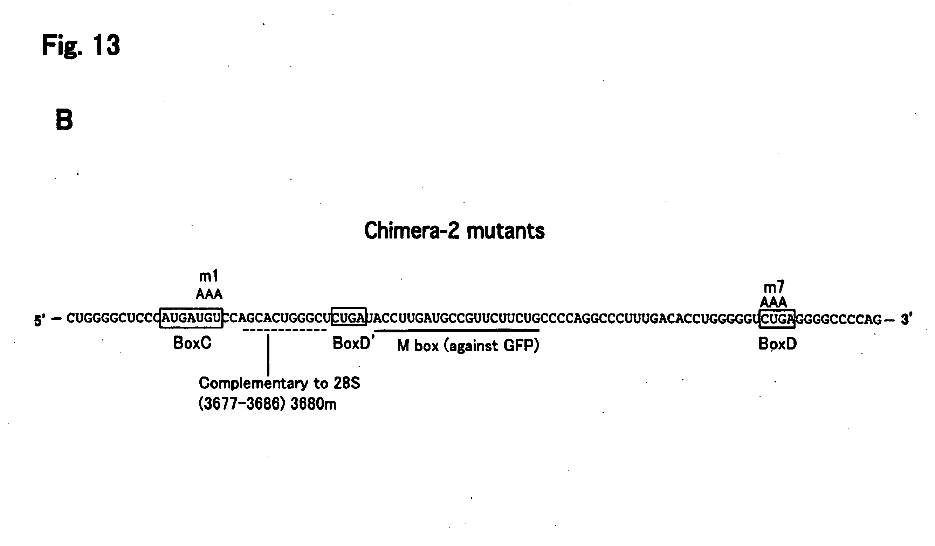

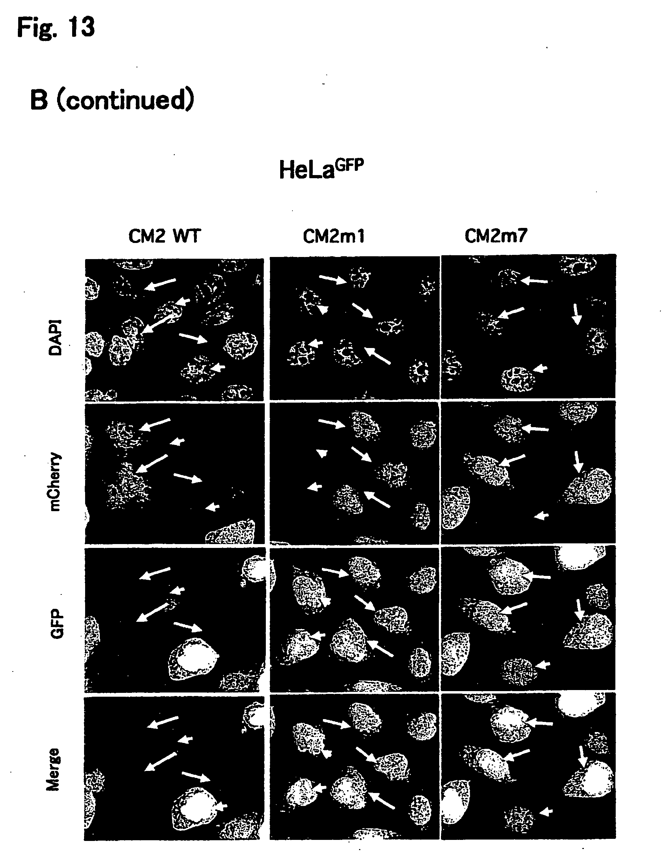

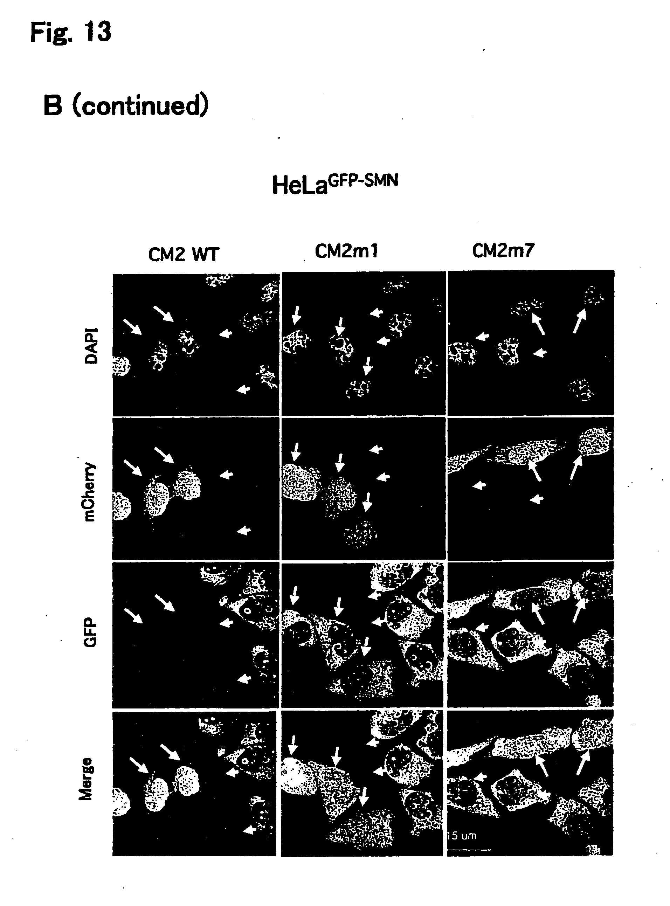

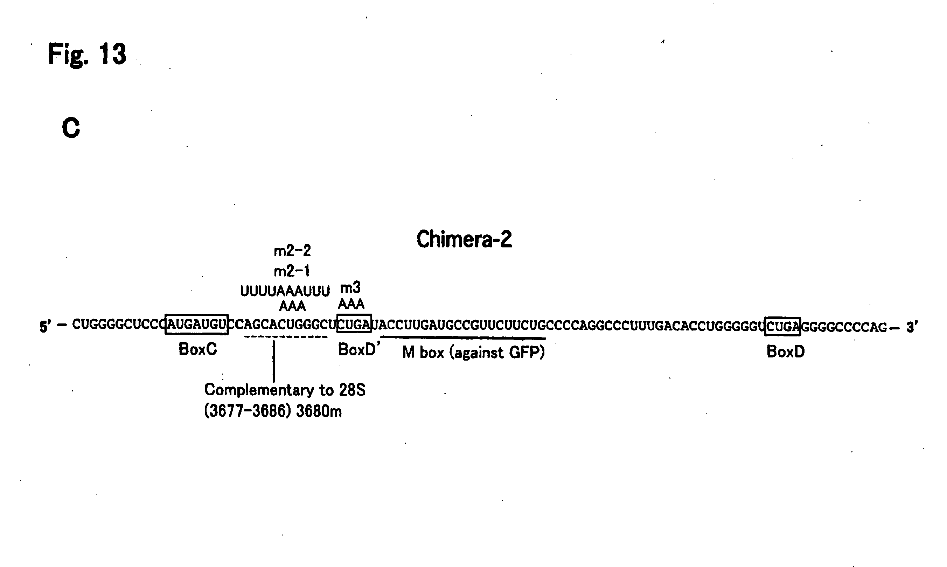

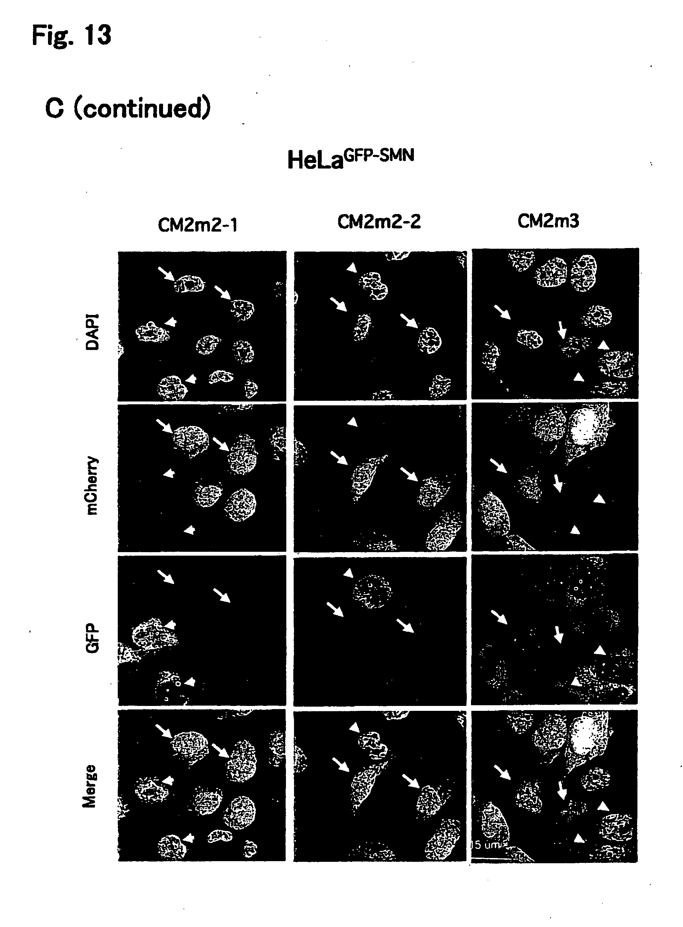

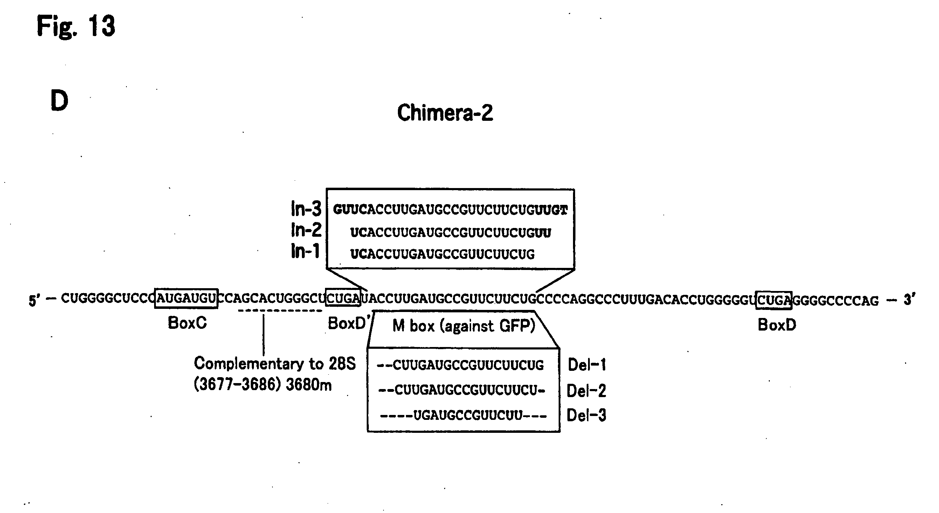

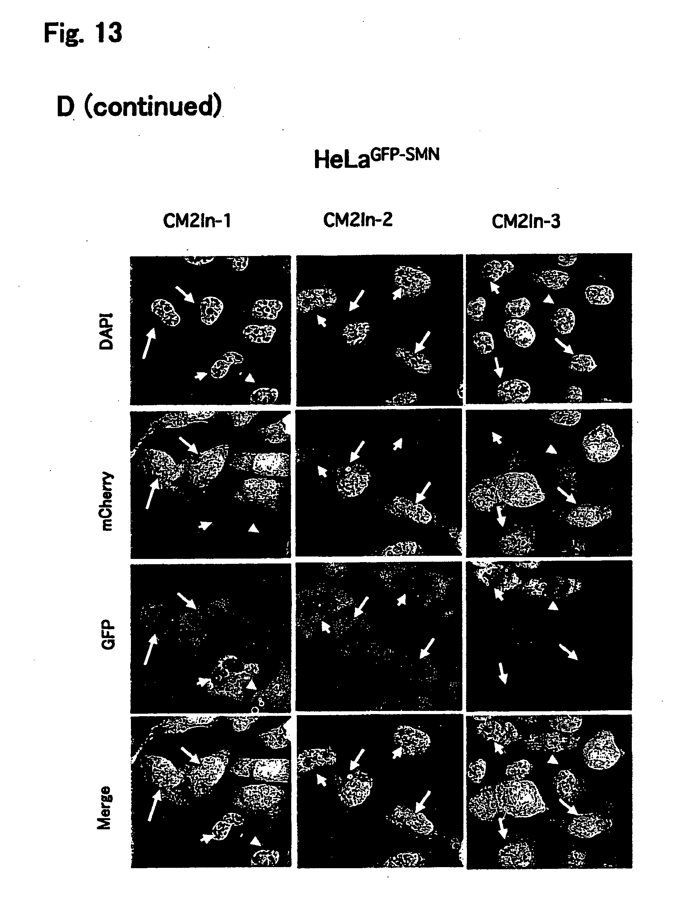

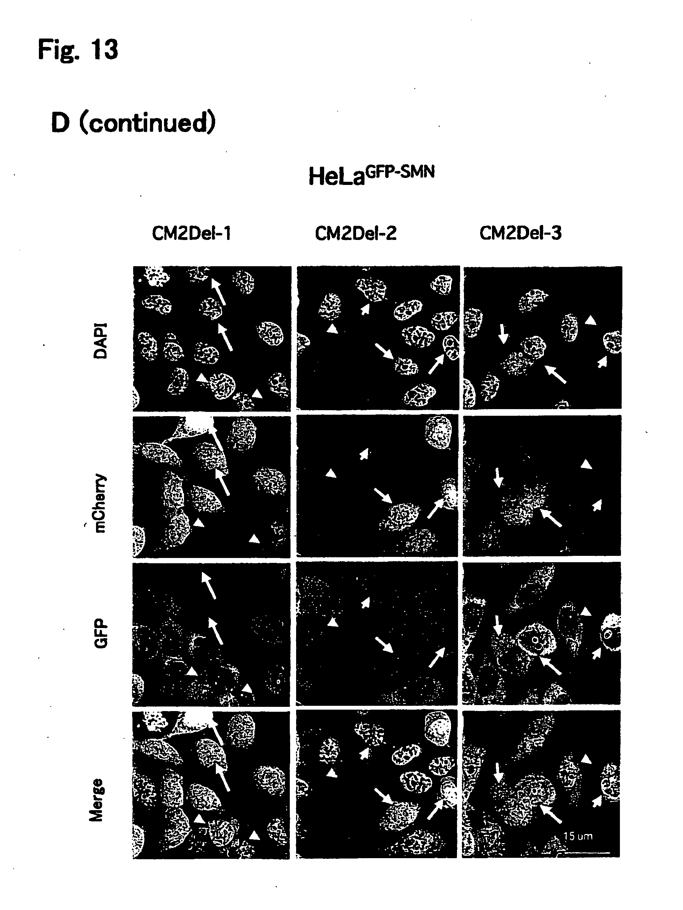

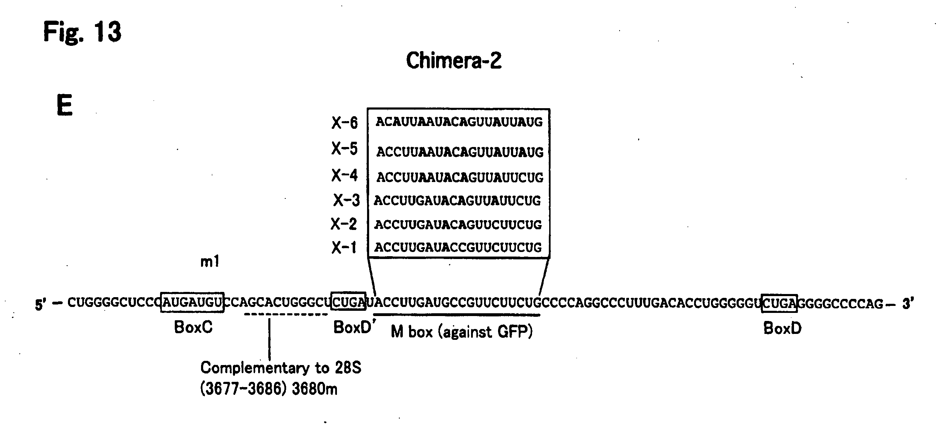

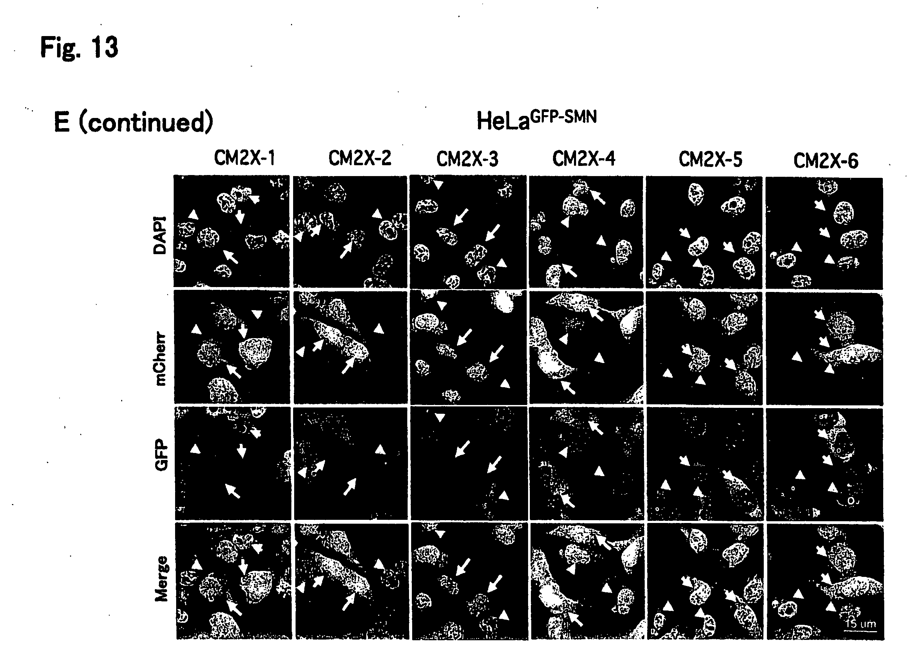

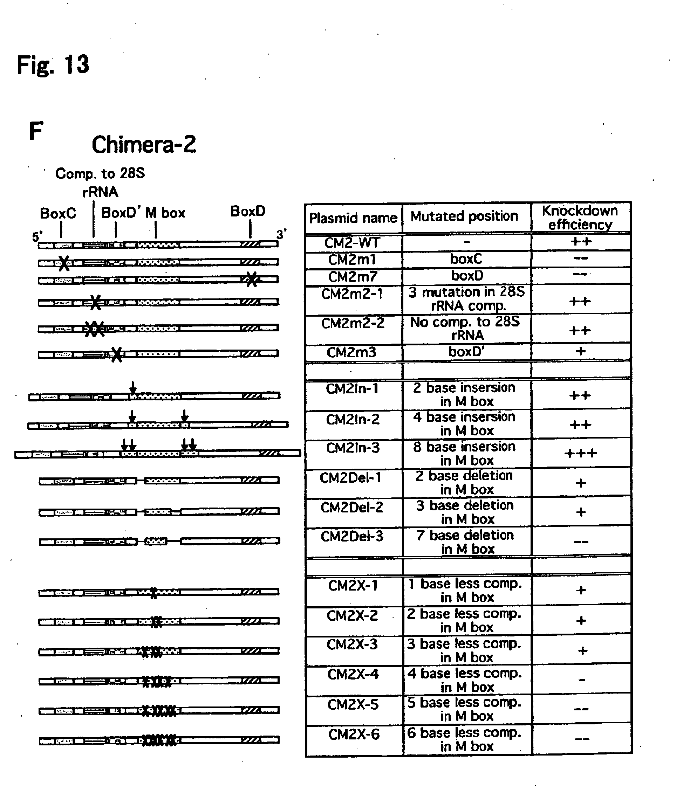

**Figure 13****: Analysis of HBII-180C and chimera snoRNA mutations.**
   (A) The sequence of snoRNA HBII-180C is shown with the positions of the eight separate three nucleotide AAA mutations indicated as m1-m8 (shown on the sequence). Box C, D & D' are indicated with boxes. The predicted guide sequence complementary to 28S rRNA is indicated by an underline, as shown in snoRNABase (L. Lestrade et al., 2006). The M box region is indicated by a bold underline. HeLa cells were transiently transfected with either an empty plasmid vector (lane 2), or with plasmid vectors expressing wild type HBII-180C (lane 3) or HBII-180C snoRNA mutants m1-8 (lanes 4-11). The same amount of total HeLa cell RNA was loaded for each lane (Ctrl: without transfection). The bands show the results of high sensitivity RNA blotting using radiolabeled oligonucleotide probes to detect HBII-180C snoRNA, and tRNA, respectively. Transfection efficiency was confirmed by semi-quantitative RT-PCR using C19 orf48 and GAPDH specific primer sets (C19orf48 and GAPDH). *The C19 orf48 primers detect a region shared between the endogenous C19orf48 transcript and the HBII-180C mini gene.
   (B) Wild type plasmid (CM2WT) and Mutant plasmids (CM2m1&m7) were transiently transfected into GFP alone stable cells (HeLa^{GFP}) or GFP-SMN fusion protein stable cell line (HeLa^{GFP-SMN}). Arrow: transfected, Arrowhead: untransfected. Note the mutations for core regions as box C (CM2m1) and box D (CM2m7) of HBII-180C snoRNA (Fig. 13A) on chimera2 plasmid showed no knockdown effect to GFP and GFP-SMN expression levels.
   (C) Mutant plasmids (CM2m2-1, m2-2 & m3) were transiently transfected into GFP-SMN fusion protein stable cell line (HeLa^{GFP-SMN}). Arrow: transfected, Arrowhead: untransfected. Note there are clear knock down effect the mutant plasmids that were destroyed 28S rRNA complementary sequence (CMm2-1 & m2-2).
   (D) Mutant plasmids that have insertions in M box to increase complementarity to GFP (CM2In-1 to -3) or deletion in M box (CM2Del-1 to -3) were transiently transfected into GFP-SMN fusion protein stable cell line (HeLa^{GFP-SMN}). Arrow: transfected, Arrowhead: untransfected.
   (E) Mutant plasmids that have point mutations in M box (CM2X-1 to -6) were transiently transfected into GFP-SMN fusion protein stable cell line (HeLa^{GFP-SMN}). Arrow: transfected, Arrowhead: untransfected.
   (F) Summary of the results of Chimera 2 mutagenesis. Left panel shows motifs of HBII-180C snoRNA and mutated regions for each plasmid (Plasmid Name and Mutated position in the table). The table shows the result of microscopy analysis (Fig. 13B-E). Knockdown efficiency shows the number of the cells that showed suppression of GFP-SMN levels as judged by counting 30 randomly selected transfected cells (+++: more than 16, ++: 13-16, +: 9-12, -: 5-8, --: less than 5).

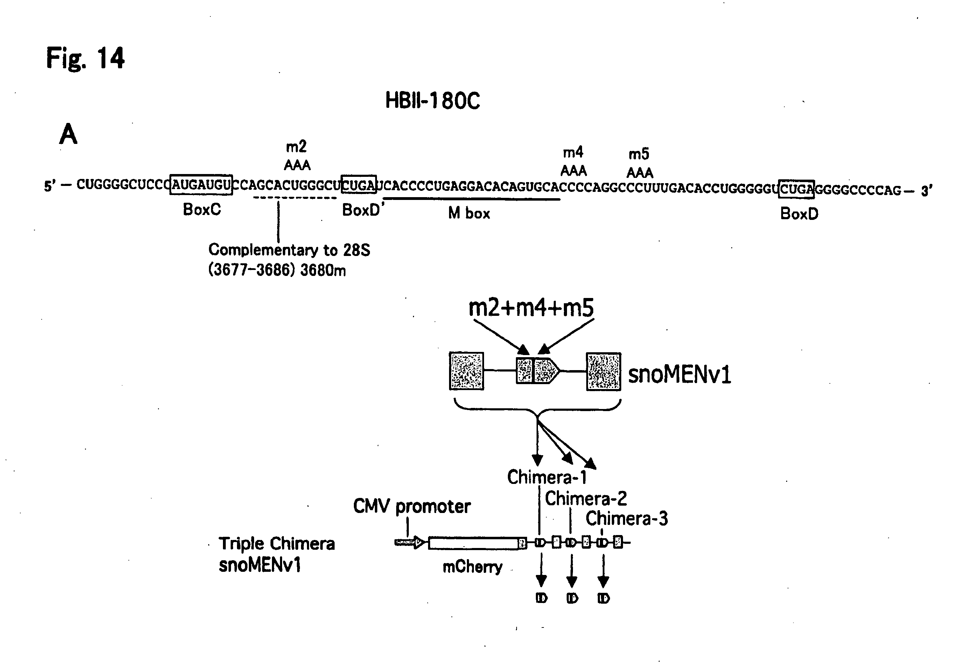

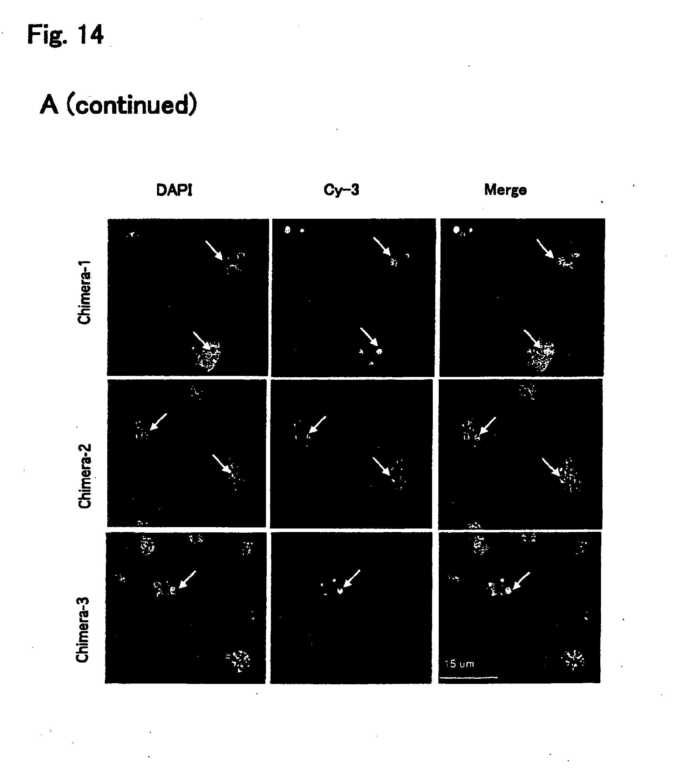

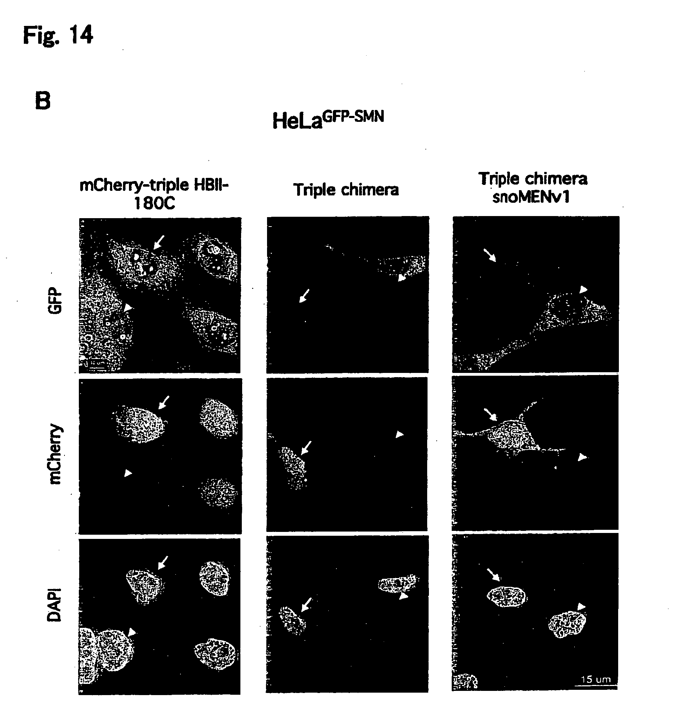

**Figure 14****: Chimera triplet plasmid incorporating combined snoRNA backbone mutations is able to mediate knockdown of expression of targeted genes.**
   (A) Mutant m2, m4 and m5 sequences were all combined and incorporated into Chimera triple snoRNA (snoMENv1). Fluorescence In Situ Hybridisation using Cy3 labelled chimera snoRNAs specific probes (Cy3) showing nucleolar localization. DNA is stained by DAPI. Bar is 15 um. Arrow shows nucleolus.
   (B) This is the same experimental design as shown in Fig.11E except that the triple chimera snoMENv1 plasmid was transfected. Note snoMENv1 plasmid showed a similar reduction level of the target gene as the wild type chimera triplet plasmid.

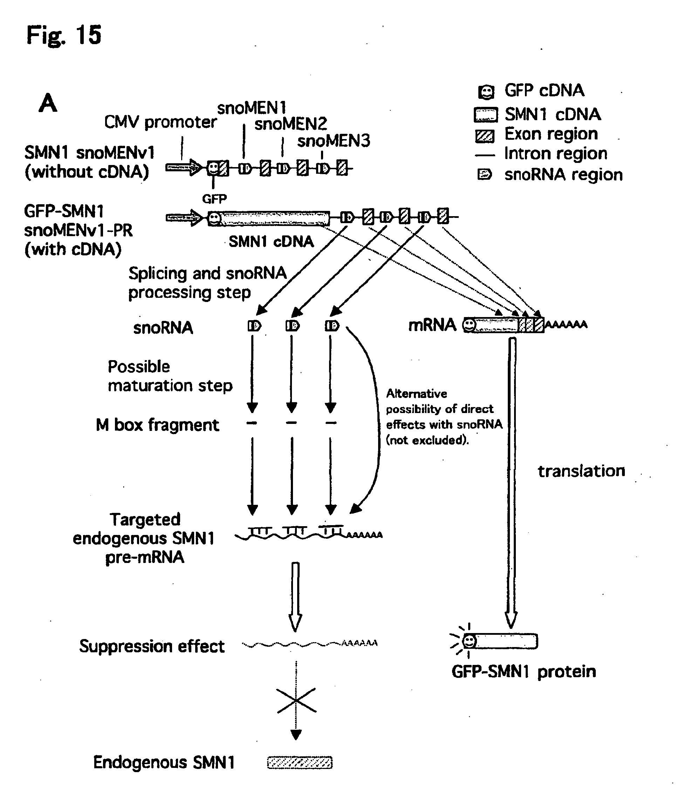

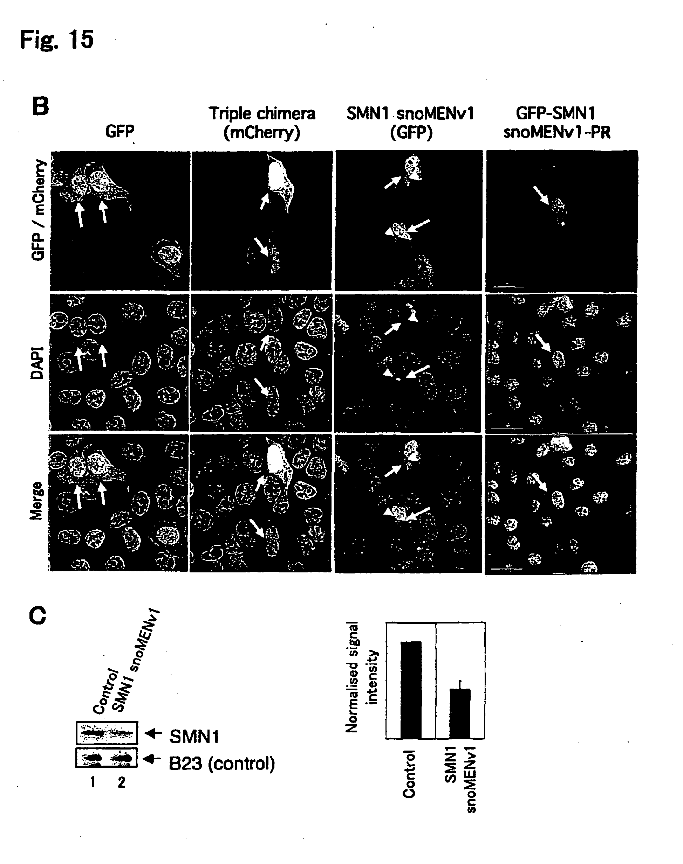

**Figure 15****: Targeted protein replacement of endogenous SMN1 protein.**
   (A) Structures for targeted endogenous SMN1 gene knockdown plasmid (SMN1 snoMENv1) and SMN1 protein replacement plasmid (GFP-SMN1 snoMENv1-PR). These constructs have a similar triple snoMEN sequence to triple chimera snoMENv1 (Fig. 14A) except that M box sequences have complementary sequences against endogenous SMN1 pre-mRNA specific sequences (See materials and Methods).
   (B) Targeted endogenous SMN1 plasmid (SMN1 snoMENv1) and protein replacement plasmid (GFP-SMN1 snoMENv1-PR) were transfected into HeLa cells. EGFP-C1 that express GFP cDNA and mcherry-triple chimera plasmid (Fig. 14A) were also transiently transfected into HeLa cells as controls (GFP & Triple chimera). Note SMN1 snoMENv1 showed cytotoxic phenotype which controls did not show. This cytotoxic effect was rescued by expression of GFP-SMN1 fusion protein using GFP-SMN1 snoMENv1-PR plasmid. Arrow: transfected cells, Arrowhead: cytotoxic phenotype cells
   (C) Detection of protein levels for endogenous SMN1 following transfection of HeLa cells using either mCherry triple chimera (Control: lane1) and SMN1 snoMENv1 (lane2). An equivalent amount of HeLa extract was loaded for each lane and the proteins separated by SDS PAGE, electroblotted and probed both with a monoclonal anti-SMN1 antibody and with anti-B23 as a loading control. Graph showed SMN1 signal intensity that have normalised by B23 signal.

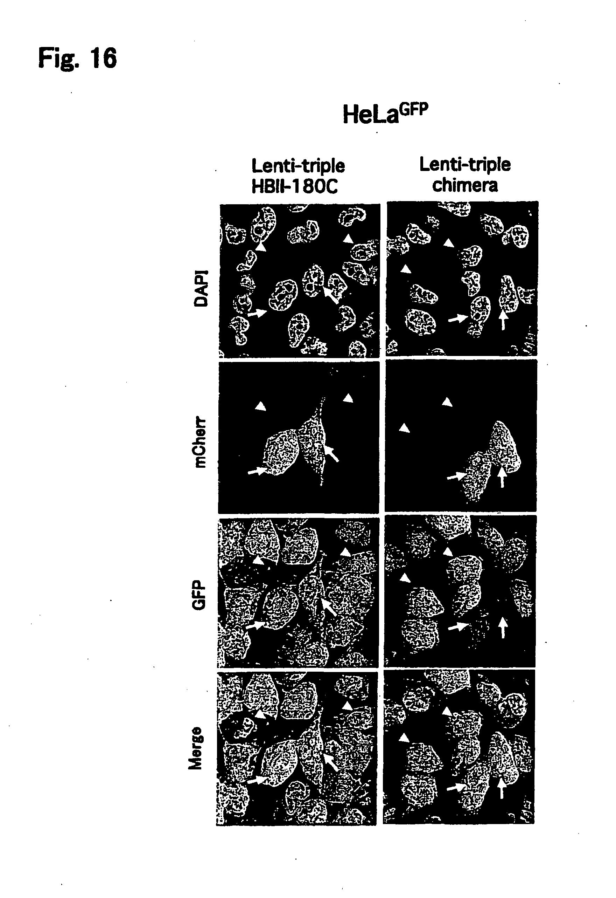

**Figure 16****: Lentiviral system with snoRNA molecule of the invention.**
   This is the same experimental design as conducted and described in Figure 11B except in this instance the expression vectors were transfected using a lentiviral vector system (Lenti-X^{™} Clontech). mCherry-triple-HBII-180C is a negative control which has 3 repeats of wild type HBII-180C snoRNA sequence in the 3' region of mCherry cDNA. Arrow: transfected cells, Arrowhead: untransfected cells.

### Abbreviations:

**snoRNA:** small nucleolar RNA
**snRNA:** small nuclear RNA
**rRNA:** ribosomal RNA
**tRNA:** transfer RNA
**FGFR3:** Fibroblast Growth Factor Receptor 3
**mRNA:** messenger RNA
**pre-mRNA:** precursor messenger RNA
**GAPDH:** glyceraldehyde phosphate dehydrogenase
**HBII-180C:** human brain snoRNA II - 180C
**CIP:** calf intestinal phosphatase
**PCR:** polymerase chain reaction
**CMV:** cytomegalovirus

### Materials and Methods

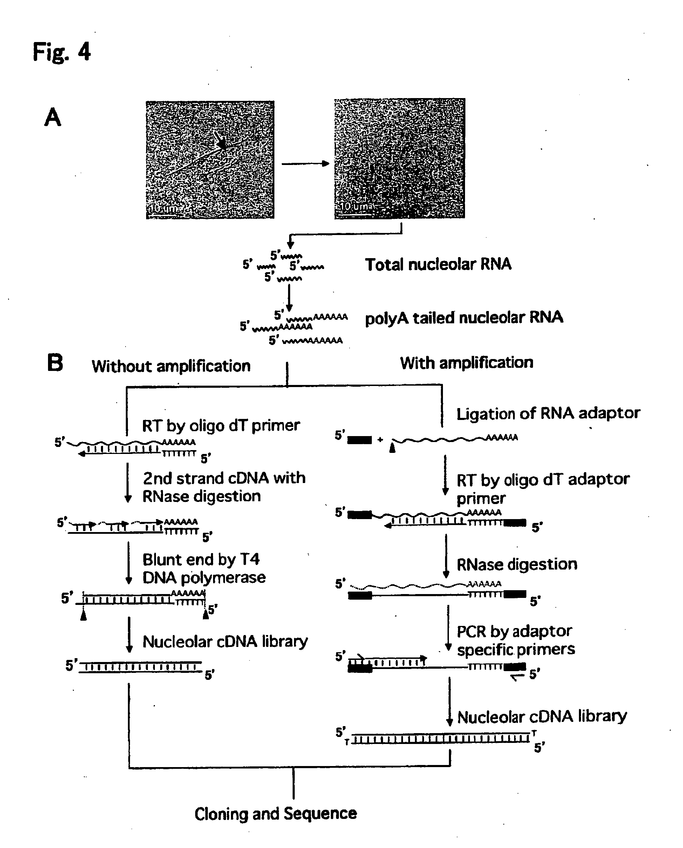

**Construction of Nucleolar cDNA library.** HeLa cell nucleoli were purified using sucrose gradients, as previously described (Andersen et. al., 2005). Nucleolar RNA was isolated from purified nucleoli by the TRIzol method with DNase I treatment according to the manufacturer's instruction (Invitrogen). Nucleolar RNA was modified by addition of a 20 to 30 mer poly adenine sequence at the 3' end using polyA polymerase (Invitrogen). In the case of the non-amplified library (Fig.4B left), cDNA was synthesized from 10 ug of polyA tailed nucleolar RNA by reverse transcription using an oligo dT primer. Second strand cDNA was synthesized by the nick translation replacement technique using the Super Script Plasmid Synthesis Kit as described by the manufacturer (Invitrogen). Double strand cDNA was cloned into pBluescript vector after creating blunt ends using T4 DNA polymerase. In the case of the amplified library (Fig. 4B right), nucleolar cDNA was made using the GeneRacer Kit without the CIP step (Invitrogen). Small size double strand cDNA was amplified by short time PCR amplification (e.g. 5 second polymerase reaction) using an adaptor specific primer as described by the manufacturer (Invitrogen). Amplified cDNA were cloned into TOPO TA cloning vector according to manufacturer's instruction.

**Northern blot analysis and RT-PCR.** Nucloelar RNA and total cell RNA were isolated from HeLa cell nucleolai and HeLa cells or human fibroblast primary cell line WI38 cells by the TRIzol method with Dnase I treatment according to manufacturer's instruction (Invitrogen). Same amount of RNA are separated by 8M Urea polyacrylamide denaturing gel in TBE buffer. The RNA was transferred onto a nylon membrane (Hybond-N; Amersham) by electro blotting. After UV cross linking, the blotted membrane was hybridyzed with ³²P labelled specific probes (U3: 5'-CACTCAGACCGCGTTCTCTCCCTCTCACTCCCCAATACGG-3',HBII-180C: 5'-AAAGGTCCTGGGGTGCACTGTGTCCTCAGGGGTGATCAGA-3'. HBII-85: 5'-ACGACGGTATGAGTTCTCACTCATTTTGTTCAGCTTTTCC-3') using standard procedures. RT-PCR was also performed to confirm the expression levels of each snoRNA. Reverse transcription and PCR were performed with gene specific primers (U3: 5'-AGAGGTAGCGTTTTCTCCTGAGCG-3' and 5'-ACCACTCAGACCGCGTTCTC-3', HBII-180C: 5'-CTCCCATGATGTCCAGCACT-3' and 5'-CTCAGACCCCCAGGTGTCAA-3', HBII-85: 5'-GATCGATGATGAGTCCCCCATAAAAAC-3' and 5'-GACCTCAGTTCTGATGAGAACGACGG-3') using SuperScript one-step RT-PCR kit (Invitrogen). To decide linearity cycles we used real time PCR using Superscript III Platinum SYBR Green one-step qRT-PCR Kit (Invitrogen) and Rotor-Gene RG-3000 system (Corbett Research). The same amount of RNA was used for RT-PCR reaction as templates. Each experiment was repeated three times independently.

**Plasmid Constructions and transfections.** The sequence from Exon 2 to 3 of the C19orf48 gene was inserted into the 3' region of the CMV promoter of the pcDNA3.1 mammalian expression plasmid creating the HBII-180C expression vector. Mutant construct (mut1, Fig.6B) was established from the wild type construct by site directed mutagenesis. The FGFR3 intron 17 expression mini gene was established to suppress endogenous HBII-180C function. Exon 17 to 18 of the FGFR3 sequence was inserted into the 3' region of CMV promoter of pcDNA3.1 mammalian expression plasmid (Fig.7A). Mutant construct (Frm, Fig.4A) was established from the wild type construct by site directed mutagenesis. The anti-GFP/YFP HBII-180C constructs (chimeral and 2, Fig8A) were established from the wild type HBII-180C expression construct by site directed mutagenesis. Mutated inserts were subcloned into the 3' region of mCherry cDNA of mammalian expression plasmid mCherry-C1 as shown in Fig.8A. The plasmids were transfected using the Calcium Phosphate method for WI38 cells and using "effectine" transfection regent (QIAGEN) for HeLa cells and other HeLa stable cell lines. The Calcium Phosphate method was performed as described below.

**Dead cell counts.** WI38 cells were spread on 10 cm dishes. The plasmids were transfected at the same time as attaching the cells. After three days the percentage of dead cells was counted five times, randomly selecting 100 cells for counting each time. The average percentage of dead cells was then calculated as the mean from the five separate counts. Each experiment was repeated 3 times independently (Fig. 8D).

**FGFR3 spliced mRNA valiant detection.** Splicing valiants of FGFR3 mRNA were detected by one-step RT-PCR using gene specific primer set (FGFR3: 5'-TGGACGTGCTGGAGCGCTCCCCGC-3' and 5'-CCCAGGGTCAGCCGGGCCCGAGACAG-3', GAPDH: 5'-CGCATCTTCTTTTGCGTCGCCAG-3' and 5'-GGTCAATGAAGGGGTCATTGATGGC-3') (Fig.7B and C). FGFR3 intron 17 expression plasmids were transfected into HeLa cells. After 48hours, the total RNA was isolated from HeLa cells, transfected HeLa cells and WI38 cells by TRIzol method with DNaseI treatment according to the manufacturer's instruction (Invitrogen). The same amount of RNA was used as templates for each RT-PCR as described above (Northern blot analysis and RT-PCR).

**Cell imaging.** All cell images were recorded using the Deltavision Spectris fluorescence microscope (Applied Precision). Live cell images for WI38, HeLa YFP-FIB cells and HeLa GFP cells were prepared as previously shown (Andersen et al., 2005, Trinkle-Mulcahy et al., 2006, http://www.lamondlab.com/f7protocols.htm). Cells were imaged using a 60X (NA 1.4) Plan Apochromat objective. Twelve optical sections separated by 0.5 µm were recorded for each field and each exposure (SoftWoRx image processing software, Aplied Precision) (Fig.4A, Fig. 6C, Fig8 C and D). Fixed cell images for WI38, HeLa YFP-FIB cells and HeLa GFP cells were prepared using the procedure previously described below.

The cells were fixed using 3.8% Paraformaldehyde. After permeabilization by 1% triton-100 cells were stained by DAPI.

### Results Section

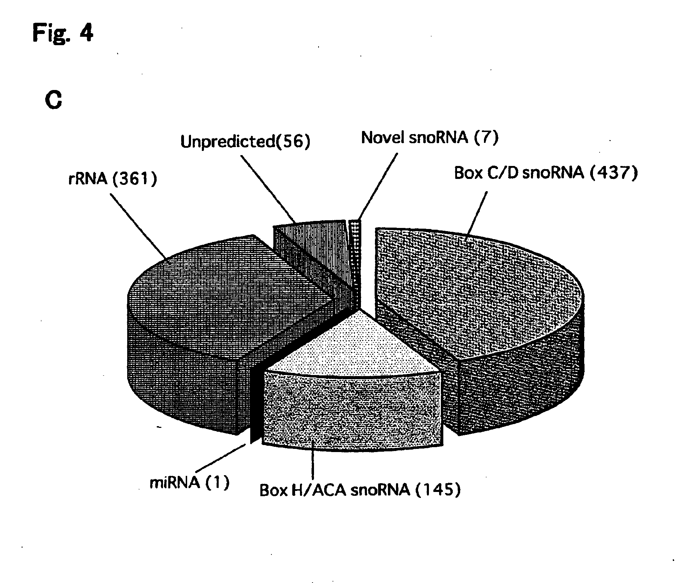

The present inventors have conducted large-scale purification and proteomic studies on nucleoli isolated from mammalian cell lines (Andersen et. al., 2002, 2005, Lam et al., 2007). They have characterized in detail the human nucleolar proteome (Leung et al., 2003, see also: http://www.lamondlab.com/NOPdb/). They have also analysed the RNA composition of nucleoli isolated from HeLa cells. RNAs were purified from highly enriched nucleolar preparations isolated from HeLa cells, size fractionated following a short time amplification reaction, subcloned (see methods in Figure 4) and sequenced. This analysis identified 589 snoRNA clones, including 562 previously known soRNAs and 7 novel snoRNAs.

Three of the known box C/D snoRNAs detected, termed HBII-180A, HBII-180B and HBII-180C, were each encoded in separate introns of a transcript termed C19orf48, (Fig.5C and D), which was previously reported to be amplified in multiple drug-resistant cancer cells (Zhou et al., 2002). Each of the three HBII-180 snoRNAs targets the same 2'-O-ribose methylation site at nucleotide position 3680 on 28S rRNA (Fig.5A and B). The gene encoding these snoRNAs is located on chromosome 19 at position q13.33. An evolutionary comparison of the C19orf48 gene sequence shows that the major conservation corresponds to the intron-encoded snoRNAs and not the respective exon sequences. This indicates that the major function of the C19orf48 gene is likely to encode snoRNAs, rather than a protein (Fig.5).

Unexpectedly, sequence analysis of the HBII-180C snoRNA revealed that it had a 21 nucleotide region, located 3' to the 28S rRNA methylation guide sequence, in which 19/21 nucleotides are complementary to a region within intron 17 of the pre-mRNA transcribed from the human Fibroblast Growth Factor Receptor 3 (FGFR3) gene (Fig.5A and B). Comparison of the expression level of HBII-180C snoRNA showed that it was highly expressed in HeLa cells but only present at low levels in primary fibroblasts (WI38 cells) (Fig.6A).

The inventors exogenously overexpressed the HBII-180C snoRNA in WI38 cells by transient transfection of a plasmid vector with a HBII-180C minigene, using the CMV promoter to drive transcription (Fig.6B). This resulted in apoptosis of the transfected WI38 cells. However, when the inventors transiently expressed a mutant of HBII-180C, with a sequence change of 3 nucleotides within the region that is complementary to intron 17 of FGFR3 (i.e., reducing complementarity to FGFR3 pre-mRNA), this no longer caused apoptosis in WI38 cells (Fig.6, B-D). In HeLa cells, overexpression from a plasmid vector of the target FGFR3 intron 17 sequence that is complementary to endogenous HBII-180C snoRNA, resulted in a clear shift in the ratio of FGFR3 mRNA isoforms expressed (Fig.7A and B). This caused an increase in HeLa cells of the short isoform of FGFR3 mRNA, resulting in a similar pattern of FGFR3 mRNA isoforms to that normally expressed in WI38 cells (Fig.7C).

These data indicate that the expression level of HBII-180C snoRNA can affect the expression levels of FGFR3 mRNA. Furthermore, the data show that this effect is dependent upon the presence in HBII-180C snoRNA of a sequence complementary to a region in intron 17 of FGFR3 pre-mRNA.

The inventors next tested whether the ability of snoRNA HBII-180C to modulate the expression of FGFR3 RNA expression could be adapted to modulate the expression of specific target RNAs by modifying the structure of HBII-180C to replace the FGFR3 complementary sequence region with sequences complementary to one or more target RNAs. Therefore a vector was constructed as shown in Figure 5A and B, called chimeral and 2, with a minigene containing the HBII-180C intron, including the HBII-I80C snoRNA backbone, expressed from a CMV promoter. The vector also encoded the mCherry fluorescent protein expressed from a CMV promoter, to allow ready detection of transected cells containing the vector. T he FGFR3 complementary region in HBII-180C was replaced with a sequence complementary to GFP RNA (see Figure 8, A and B). The inserted sequences were 5'-GACTTGAAGAAGTCGTGCTGC-3' (chimeral) and 5'-ACCTTGATGCCGTTCTTCTGC-3' (chimera2), which replaced the sequence 5'-CACCCCTGAGGACACAGTGCA-3' in wt HBII-180C.

The chimera expression vectors and equivalent vector expressing mCherry alone (mCherry-C1), were each transiently transfected into either a/ a HeLa cell line stably expressing unfused GFP alone or b/ a HeLa cell line stably expressing YFP fused with fibrillarin (Fig.8, C and D). Cells were fixed at 48 hours post transfection and the level of GFP/YFP and mCherry expression analysed by fluorescence microscopy. This showed that the level of GFP fluorescence in the respective HeLa stable cell lines was specifically reduced in those cells transfected with the vector engineered to contain a sequence complementary to GFP within the HBII-180C snoRNA backbone. This reduction was only observed in cells expressing mCherry, confirming that it occurred specifically in transfected cells. In contrast, cells transfected with the mCherry vectors lacking a snoRNA did not show a significant reduction in GFP fluorescence, or a variation in the level of GFP between cells expressing mCherry and those not expressing mCherry (Fig.8C& D). The data show that expression of either GFP alone, or a fusion protein formed by the closely related YFP and a cell protein (fibrillarin), can both be modulated through the expression of specifically targeted, engineered snoRNA expression vector.

These data show that the sequence and structure of the snoRNA expression vector the inventors have created can be engineered to target a specific RNA target of choice and thereby modulate its expression.

### Further Materials and Methods

**Northern and High sensitivity RNA blot analysis.** HeLa cell extracts were fractionated using sucrose gradients, as previously described. Total HeLa cell RNA and RNA from separate cytoplasmic, nucleoplasmic and nucleolar fractions was isolated using the TRIzol method, with Dnase I treatment, according to manufacturer's instruction (Invitrogen). Equal amounts of RNA from each sample were separated by 8M Urea polyacrylamide denaturing gel electrophoresis in 1xTBE buffer and the RNA transferred onto nylon membrane (Hybond-N; Amersham) by electro blotting. After UV cross linking, the membrane was hybridized with ³²P 5' end-labelled oligoribonucleotide probes specific for the following RNA species; (HBII-180A, HBII-180B, HBII-180C & tRNA). High sensitivity RNA blots were prepared as previously described (G. S. Pall et al., 2007).

### Secondary structure

All RNA secondary structures were predicted by RNAstructure 4.5 (D. H. Mathews et al., 2004) and annotated using RnaViz 2.0 (P. De Rijk et al., 2003).

### Plasmid Construction and transfections

The sequence from Exon 2 to 3 of the C19 orf48 gene was inserted 3' of the CMV promoter in the pcDNA3.1 mammalian expression plasmid (Invitrogen), creating the HBII-180C expression vector. HBII-180A, B and mutant constructs of HBII-180C were established from the wild type HBII-180C expression mini gene construct by site directed mutagenesis. The plasmids were transfected into HeLa cells using "effectine" transfection regent (QIAGEN).

### Cell imaging

All cell images were recorded using the Deltavision Spectris fluorescence microscope (Applied Precision). Live cell images for HeLa^{YFP- Fibrillarin} cells and HeLa^{GFP} cells were prepared as previously shown (L. Trinkle-Mulcahy et al., 2006 and K. L. Leung et. al., 2004). (http://www.lamondlab.com/f7protocols.htm). Cells were imaged using a 60X (NA 1.4) Plan Apochromat objective. Twelve optical sections separated by 0.5 µm were recorded for each field and each exposure (SoftWoRx image processing software, Applied Precision).

### Results Section

### Modified snoRNA effect on targeted protein and/or mRNA expression levels

Northern blot analysis showed that transient transfection of chimera snoRNA targeted to G/YFP mRNA sequence suppressed G/YFP mRNA levels (Fig. 9C & D). This result suggested that chimera snoRNA can influence target mRNA expression levels as described (Fig. 1 &2).
Multiple modified snoRNAs can be expressed in one transcript to increase their efficiency.

The triplet chimera snoRNA construct was established as in figure 11A. This plasmid (Triple chimera) encodes three modified snoRNAs which target different positions of in the G/YFP mRNA sequence 3' to the mCherry fluorescence protein cDNA which is used here as a transfection marker (Fig.11A). A transient transfection experiment using this triple chimera plasmid showed a similar suppression effect to G/YFP and G/YFP-fusion proteins as seen with a single chimera plasmids as judged by fluorescence microscopy (Fig. 11B, C, E). The western blot analysis showed that the suppression efficiency of the triple chimera construct was stronger than the single chimera snoRNAs (Fig. 11D). FISH analysis and fractionated northern blot analysis using specific probes for each chimera snoRNA showed that the triplet chimera plasmid generated each of the modified chimera 1-3 snoRNAs as seen with wild type HBII-180C (Fig. 12A & B). These results suggested that modified snoRNA can increase efficiency of targeted knock down by expressing multiple snoRNAs in one transcript (multiplexing). Moreover, the triple chimera plasmid expressed mCherry red fluorescence protein instead of G/YFP and G/YFP fusion proteins (Fig. 11B, C, E). These results also suggested that we could replace the G/YFP and G/YFP-fusion protein with mCherry by using modified snoRNA, thus demonstrating a "protein replacement" or "protein knock-in" effect.

Detailed structural analysis for modified snoRNA and their knockdown ability.
The knockdown ability of modified snoRNA could either arise from processed snoRNA, or alternatively could be formed independently of snoRNA processing directly from the C9orf48 pre-mRNA (Fig. 5C). The latter possibility is suggested by the recent reports of intron-encoded micro RNAs processed independendy of Drosha. To distinguish between these possibilities, a series of three base mutations were introduced at eight separate regions of an HBII-180C minigene and the expression of both full length snoRNA compared between the WT and mutant forms of HBII-180C (Fig. 13A). Expression of the WT HBII-180C minigene vector elevates the level of full length HBII-180C snoRNA over endogenous levels, but the empty vector does not (Fig. 13A, compare lane 1 with lanes 2 & 3). Multiple mutations, including sequence changes within either of the conserved C or D boxes, reduce or eliminate expression of exogenous full length HBII-180C snoRNA, leaving only the endogenous level of snoRNA (Fig. 13A, lanes 4-11). In each case where the level of HBII-180C expression is reduced, there is a corresponding decrease in the level of knockdown ability of modified snoRNA chimera-2 (Fig. 13A, compare lane 3 with lanes 4 and 10, and Fig. 13B m1 and m7, Fig. 13F). This indicates that the knockdown ability of modified snoRNA is dependent upon prior snoRNA processing and argues that there is likely not a snoRNA-independent pathway for knockdown ability directly from the C19orf48 pre-mRNA transcript. The m2 mutation in HBII-180C, which destroys the complementarity to 28S rRNA in the snoRNA guide sequence, does not prevent snoRNA formation and knockdown efficiency of modified snoRNA (Fig. 13A, lane 5, Fig. 13C & F m2-1, m2-2). Therefore, the ability of modified snoRNA to knock down expression of target genes does not depend upon the ability of the snoRNA to bind to and methylate rRNA. The m3 mutation in HBII-180C, which destroys the box D', slightly decrease prevent snoRNA formation and knockdown efficiency (Fig. 13A, C, F). This suggested that we might modify the knockdown ability of modified snoRNA by mutating the region outside of the M box.

The insertion and deletion mutants of the M box sequence in modified snoRNA chimera-2 were established to test the association between knockdown ability and M box sequence length (Fig. 13D & F). All three insertion mutants which increased sequence complementary to G/YFP mRNA showed similar knockdown activity to the wild type chimera-2 plasmid (Fig 13D & F In1-3). Remarkably, insertion mutant CM2In-3 that has 8 base additional complementary sequence to G/YFP mRNA showed enhanced knockdown efficiency (Fig. 13F). The 2 and 3 base deletion mutants in M box (CM2Del-1 & -2) still showed knockdown ability but it was less efficient compared with wild type chimera-2 (Fig.13F). The 7 base deletion mutants CM2Del-3 didn't show significant knockdown activity as compared with wild type Chimera-2 (Fig. 13F). The 6 point mutants in M box were also established to test association between knockdown ability and base pare mismatch in M box (Fig. 13E & F). The 1-3 base mismatch mutants in M box (CM2X-1 to -3) still showed knockdown ability but it was less efficient compared with wild type chimera-2 (Fig.13F). The 4-6 base mismatch mutants in M box (CM2X-4 to -6) didn't show efficient knockdown activity compared with wild type chimera-2 (Fig. 13F). These results suggested that the ability of modified snoRNA to modulate gene knock down is associated with the hybridization affinity between the M box complementary and target mRNA sequences.

The combination snoRNA incorporating each of the mutants for m2 (28S rRNA complementary region), m4 and m5 that are not expected to affect modified snoRNA knockdown ability (Fig. 13A & C, Fig. 14A) showed similar knockdown ability as the wild type triple chimera plasmid as judged by fluorescence microscopy (Fig. 14B). FISH analysis using chimera-1, -2, -3 specific probes showed that this combination mutant plasmid (triple chimera snoMENv1) express each of the encoded chimera modified snoRNAs as also seen with the wild type triple chimera plasmid (Fig. 12A & 14A). These mutant analyses suggested that the knockdown ability of modified snoRNAs can be modulated both by mutations lying outside the sequence that is associated with snoRNA binding to the target RNA and mutations lying within the complementary sequence (ie M Box) that is required for binding between the snoRNA and target sequence.

### Modified snoRNA vector can rescue a lethal gene expression knockdown effect by protein replacement.

The two modified snoRNA plasmids targeted to endogenous SMN1 (Survival of Motor Neurons) pre-mRNA were established from triple chimera snoMENv1 (Fig.15A). Plasmid SMN1 snoMENv1 encodes GFP cDNA and 3 modified snoRNAs targeted to different positions within endogenous SMN1 pre-mRNA, respectively (Fig. 15A). The other plasmid GFP-SMN1 snoMENv1-PR encodes GFP-SMN1 fusion protein cDNA and 3 same modified snoRNAs with SMN1 snoMENv1 targeted to different positions within endogenous SMN1 pre-mRNA, respectively (Fig. 15A). Transient transfection analysis showed that GFP alone and mCherry triple chimera plasmid had no deleterious effect on HeLa cells. However, the SMN1 snoMENv1 plasmid showed a cytotoxic effect when transfected into cells (Fig. 15B arrowhead). Western blot analysis showed that the endogenous SMN1 level was suppressed by transfecting SMN1 snoMENv1 (Fig. 15C). This result was expected because the phenotype of SMN1 knock out mice is known to be embryonic lethal (Hsieh et al, Nature Genetics 2000). On the other hand, transient transfection of GFP-SMN1 snoMENv1-PR plasmid showed the correct localization pattern as seen in the HeLa^{GFP-SMN1} stable cell line (Fig. 10) and, importantly, did not show a cytotoxic effect as seen with SMN1 snoMENv1 (Fig. 15B). These results indicated that endogenous SMN1 was suppressed by the modified snoRNAs targeted to SMN1 pre-mRNA and their cytotoxic effect was rescued by expressing the GFP-SMN1 fusion protein.

### Modified snoRNA function when expressed from a viral vector system.

Triple chimera modified snoRNA (lenti-triple chimera) and wild type triple HBII-180C snoRNA (lenti-triple HBII-180C) were each subcloned into lenti-X viral vector (Clontech). The lenti-triple chimera showed the same knockdown effect as seen using the corresponding plasmid vector triple chimera (compare Fig. 11B & Fig. 16). This result showed that the ability of modified snoRNA to knock down targeted genes is not dependent on a single transfection system and can be delivered using both plasmid and viral vector systems.

### REFERENCES:

1. T. Kiss, Cell 109, 145 (2002).
2. T. Kiss et al, Cold Spring Harb Symp Quant Biol. 71,407-17 (2006).
3. F-M. Boisvert et. al., Nat Rev Mol Cell Biol. 8, 574-585 (2007).
4. A.K.L., Leung et. al., Biochem. J., 376, 553-569 (2003).
5. S. Kishore and S. Stamm, Science, 311, 230 (2006).
6. JS. Andersen et al., Curr Biol., 12, 1-11 (2002).
7. JS. Andersen et al., Nature, 433, 77-83 (2005).
8. YW. Lam et al., Curr. Biol. 17, 749-60 (2007).
9. XD. Zhou et al.,Ai zheng 4, 341-5 (2002).
10. L. Trinkle-Mulcahy et al., J. Cell Biol. 172, 679-92 (2006).
11. A.K.L., Leung et. al., J Cell Biol. 166, 787-800 (2004).
12. G. S. Pall et al., Nucleic Acids Res. 35 (8), e60 (2007).
13. D. H. Mathews et al., Proc. Natl. Acad. Sci. U S A 101 (19), 7287-92 (2004).
14. P. De Rijk et al., Bioinformatics 19 (2), 299-300 (2003).
15. L. Trinkle-Mulcahy et al., J. Cell Biol. 172, 679-92 (2006).
16. K. L. Leung et. al., J. Cell Biol. 166, 787-800 (2004).
17. L. Lestrade et al., Nucleic Acids Res. 34, D158-162 (2006).
18. Maden, B. E. H. Prog. Nuc. Ac. Res. Mol. Biol. 39, 241-303 (1990).
19. Lestrade, L. & Weber, W. J. Nucleic Acids Res., 34, D158-162 (2006)..
20. M. Zuker, Nucleic Acids Res. 31 (13), 3406-15, (2003)
20. D.H. Mathews, J. Sabina, M. Zuker and D.H., J. Mol. Biol. 288, 911-940 (1999)
21. Kent, W. J. et al., Genome Res. 12 (6), 996-1006 (2002).
22. Glover, D., et al., Nature Reviews, Genetics, 6, 299-311 (2005).

## Claims

1. A method of modulating expression of a target nucleic acid, the method comprising contacting the nucleic acid with a snoRNA or modified snoRNA under conditions whereby the snoRNA or modified snoRNA and/or fragment thereof is capable of hybridising to a portion of the target nucleic acid; and wherein hybridisation of the snoRNA or fragment thereof to said portion of nucleic acid modulates expression of the target nucleic acid.

2. A modified snoRNA sequence, the modified snoRNA sequence comprising a nucleic acid sequence substantially complementary to a portion of the target sequence, for hybridising to said portion of the target nucleic acid and wherein hybridisation of the modified snoRNA sequence to said target sequence modulates expression of the target nucleic acid.

3. The method or modified snoRNA according to any preceding claim wherein the sequence substantially complementary to a portion of target RNA sequence is substantially complementary to either an intron or exon sequence, or to an intron - exon junction sequence, or to a region within or at the junction of the 5' or 3' untranslated region of said target nucleic acid.

4. The method or modified snoRNA according to any preceding claim wherein the nucleic acid sequence which is substantially complementary to said portion of target RNA sequence, typically 15-45 nucleotides in length.

5. A nucleic acid construct, including plasmid and viral vectors, capable of expressing at least one modified snoRNA molecule according to any of claims 2 - 4.

6. The nucleic acid construct according to claim 5 comprising at least two regions of exonic nucleic acid flanking a region of intronic nucleic acid, which is capable of encoding said at least one modified snoRNA.

7. The nucleic acid construct according to either of claims 5 or 6 wherein the nucleic acid construct comprises a multiplicity of exonic sequences flanking two or more intronic sequences comprising sequence capable of encoding one or more snoRNAs

8. A host cell transformed or transfected with a nucleic acid construct according to either of claims 5 - 7.

9. A nucleic acid vector construct for use in generating a modified snoRNA molecules, the construct comprising in a 5' to 3' direction
i) a promoter sequence for controlling transcription;
ii) a first exon sequence;
iii) a first intron splicing sequence;
iv) a cloning site or sequence for facilitating cloning of a nucleic acid sequence encoding a modified snoRNA according to any of claims 2 - 3 ;
v) a second intron splicing sequence; and
vi) a second exon sequence.

10. The nucleic acid vector construct according to claim 9, further comprising in a 5' to 3' direction
vii) a third or further intron splicing sequence;
vii) a second or further cloning site or sequence for facilitating cloning of a nucleic acid sequence encoding a modified snoRNA according to any of claims 2 - 3;
viii) a fourth or further intron splicing sequence; and
ix) a third or further exon sequence.

11. The nucleic acid vector construct according to claims 5 - 7, 9 or 10, further comprising an expressible coding sequence encoding a protein, RNA, tagged protein, mutant protein; or the like, to functionally replace the target RNA, or protein to be modulated by said one or more snoRNAs.

12. A snoRNA, modified snoRNA and/or fragment thereof, capable of hybridising to a portion of target nucleic acid, or nucleic acid construct encoding a snoRNA, or modified snoRNA and/or fragment thereof, capable of hybridising to a portion of target nucleic acid, or cell capable of expressing a snoRNA or modified snoRNA and/or fragment thereof, capable of hybridising to a portion of target nucleic acid, or for use in treating a disease or a condition in a subject where it is desirable to modulate, especially down-regulate expression of a target nucleic acid.

13. A pharmaceutical composition comprising a snoRNA, modified snoRNA and/or fragment thereof, capable of hybridising to a portion of target nucleic acid, or nucleic acid construct encoding a snoRNA or modified snoRNA and/or fragment thereof, capable of hybridising to a portion of target nucleic acid, or cell capable of expressing a snoRNA or modified snoRNA and/or fragment thereof, capable of hybridising to a portion of target nucleic acid, and a pharmaceutically acceptable carrier therefore.

14. The product or composition according to either of claims 12 or 13, comprising a modified snoRNA, nucleic acid construct encoding a snoRNA or modified snoRNA, or cell capable of expressing a snoRNA or modified snoRNA, according to any of claims 2-11.

15. Use of a vector according to claims 5 - 7, 9 or 10 for assessing mutant phenotypes, or to aid drug screening strategies, or for target validation, or to analyse a tagged form of a protein without competition from the existing, untagged cellular form, or for other applied or research applications.

## Patentansprüche

1. Verfahren zum Modulieren der Expression einer Zielnucleinsäure, wobei das Verfahren Inkontaktbringen der Nucleinsäure mit einer snoRNA oder modifizierten snoRNA unter Bedingungen umfasst, wodurch die snoRNA oder modifizierte snoRNA und/oder ein Fragment davon fähig ist, mit einem Anteil der Zielnucleinsäure zu hybridisieren; und wobei Hybridisierung der snoRNA oder eines Fragments davon mit dem Anteil der Nucleinsäure die Expression der Zielnucleinsäure moduliert.

2. Modifizierte snoRNA-Sequenz, wobei die modifizierte snoRNA-Sequenz eine Nucleinsäuresequenz, im Wesentlichen komplementär zu einem Anteil der Zielsequenz, umfasst, zum Hybridisieren mit dem Anteil der Zielnucleinsäure und wobei Hybridisierung der modifizierten snoRNA-Sequenz mit der Zielsequenz die Expression der Zielnucleinsäure moduliert.

3. Verfahren oder modifizierte snoRNA gemäß einem vorhergehenden Anspruch, wobei die Sequenz, im Wesentlichen komplementär zu einem Anteil der Ziel-RNA-Sequenz, im Wesentlichen komplementär zu entweder einer Intron- oder einer Exon-Sequenz oder zu einer Intron-Exon-Verbindungssequenz oder zu einer Region innerhalb oder an der Verbindung der 5'- oder 3'-untranslatierten Region der Zielnucleinsäure ist.

4. Verfahren oder modifizierte snoRNA gemäß einem vorhergehenden Anspruch, wobei die Nucleinsäuresequenz, welche im Wesentlichen komplementär zu dem Anteil der Ziel-RNA-Sequenz ist, typischerweise eine Länge von 15-45 Nucleotiden hat.

5. Nucleinsäurekonstrukt, einschließend Plasmid- und virale Vektoren, fähig zum Exprimieren von mindestens einem modifizierten snoRNA-Molekül gemäß einem der Ansprüche 2-4.

6. Nucleinsäurekonstrukt gemäß Anspruch 5, umfassend mindestens zwei Regionen von exonischer Nucleinsäure, flankierend eine Region von intronischer Nucleinsäure, das fähig ist, die mindestens eine modifizierte snoRNA zu codieren.

7. Nucleinsäurekonstrukt gemäß einem der Ansprüche 5 oder 6, wobei das Nucleinsäurekonstrukt eine Vielzahl von exonischen Sequenzen, flankierend zwei oder mehrere intronische Sequenzen, umfassend eine Sequenz, fähig zum Codieren von einer oder mehreren snoRNAs, umfasst.

8. Wirtszelle, transformiert oder transfiziert mit einem Nucleinsäurekonstrukt gemäß einem der Ansprüche 5-7.

9. Nucleinsäurevektor-Konstrukt zur Verwendung beim Generieren von modifizierten snoRNA-Molekülen, wobei das Konstrukt in einer 5'- nach 3'-Richtung umfasst
i) eine Promotorsequenz zum Kontrollieren der Transkription;
ii) eine erste Exon-Sequenz;
iii) eine erste Intron-Splicing-Sequenz;
iv) eine Klonierungsstelle oder -sequenz zum Erleichtern der Klonierung einer Nucleinsäuresequenz, codierend eine modifizierte snoRNA gemäß einem der Ansprüche 2-3;
v) eine zweite Intron-Splicing-Sequenz; und
vi) eine zweite Exon-Sequenz.

10. Nucleinsäurevektor-Konstrukt gemäß Anspruch 9, weiterhin umfassend in einer 5'- nach 3'-Richtung
vii) eine dritte oder weitere Intron-Splicing-Sequenz;
vii) eine zweite oder weitere Klonierungsstelle oder -sequenz zum Erleichtern der Klonierung einer Nucleinsäuresequenz, codierend eine modifizierte snoRNA gemäß einem der Ansprüche 2-3;
viii) eine vierte oder weitere Intron-Splicing-Sequenz; und
ix) eine dritte oder weitere Exon-Sequenz.

11. Nucleinsäurevektor-Konstrukt gemäß den Ansprüchen 5-7, 9 oder 10, weiterhin umfassend eine exprimierbare codierende Sequenz, codierend ein Protein, RNA, markiertes Protein, mutantes Protein oder dergleichen, um funktionell die Ziel-RNA, oder das Protein, das durch die eine oder die mehreren snoRNAs moduliert werden soll, zu ersetzen.

12. snoRNA, modifizierte snoRNA und/oder Fragment davon, fähig zum Hybridisieren mit einem Anteil einer Zielnucleinsäure, oder Nucleinsäurekonstrukt, codierend eine snoRNA oder modifizierte snoRNA und/oder ein Fragment davon, fähig zum Hybridisieren mit einem Anteil einer Zielnucleinsäure, oder Zelle, fähig zum Exprimieren einer snoRNA oder modifizierten snoRNA und/oder eines Fragments davon, fähig zum Hybridisieren mit einem Anteil einer Zielnucleinsäure, oder zur Verwendung beim Behandeln einer Krankheit oder eines Leidens bei einem Patienten, wo es wünschenswert ist, die Expression einer Zielnucleinsäure zu modulieren, speziell herabzuregulieren.

13. Pharmazeutische Zusammensetzung, umfassend eine snoRNA, modifizierte snoRNA und/oder ein Fragment davon, fähig zum Hybridisieren mit einem Anteil einer Zielnucleinsäure, oder Nucleinsäurekonstrukt, codierend eine snoRNA oder modifizierte snoRNA und/oder ein Fragment davon, fähig zum Hybridisieren mit einem Anteil einer Zielnucleinsäure, oder Zelle, fähig zum Exprimieren einer snoRNA oder modifizierten snoRNA und/oder eines Fragments davon, fähig zum Hybridisieren mit einem Anteil einer Zielnucleinsäure, und einen pharmazeutisch verträglichen Träger dafür.

14. Produkt oder Zusammensetzung gemäß einem der Ansprüche 12 oder 13, umfassend eine modifizierte snoRNA, Nucleinsäurekonstrukt, codierend eine snoRNA oder modifizierte snoRNA, oder Zelle, fähig zum Exprimieren einer snoRNA oder modifizierten snoRNA gemäß einem der Ansprüche 2-11.

15. Verwendung eines Vektors gemäß den Ansprüchen 5-7, 9 oder 10 zum Bewerten mutanter Phänotypen oder zum Unterstützen von Arzneimittel-Screening-Strategien oder zur Zielvalidierung oder zum Analysieren einer markierten Form eines Proteins ohne Konkurrenz von der existierenden, unmarkierten zellulären Form oder für andere angewandte oder Forschungsanwendungen.

## Revendications

1. Procédé de modulation de l'expression d'un acide nucléique cible, le procédé comprenant mettre l'acide nucléique en contact avec un snoARN ou un snoARN modifié dans des conditions par lesquelles le snoARN ou snoARN modifié et/ou fragment de celui-ci est capable de s'hybrider à une portion de l'acide nucléique cible; et où l'hybridation du snoARN ou fragment de celui-ci à ladite portion d'acide nucléique module l'expression de l'acide nucléique cible.

2. Séquence de snoARN modifié, la séquence de snoARN modifié comprenant une séquence d'acide nucléique sensiblement complémentaire à une portion de la séquence cible pour hybridation à ladite portion de l'acide nucléique cible et où l'hybridation de la séquence de snoARN modifié à ladite séquence cible module l'expression de l'acide nucléique cible.

3. Procédé ou snoARN modifié selon l'une quelconque des revendications précédentes, où la séquence sensiblement complémentaire à une portion de la séquence d'ARN cible est sensiblement complémentaire à soit une séquence intron soit une séquence exon ou à une séquence de jonction intron-exon, ou à une région dans/ou à la jonction de la région non traduite en 5' ou en 3' dudit acide nucléique cible.

4. Procédé ou snoARN modifié selon l'une quelconque des revendications précédentes, où la séquence d'acide nucléique qui est sensiblement complémentaire à ladite portion de la séquence d'ARN cible, fait typiquement 15-45 nucléotides de long.

5. Produit de synthèse d'acide nucléique, incluant des vecteurs plasmidiques et viraux capables d'exprimer au moins une molécule de snoARN modifié selon l'une quelconque des revendications 2-4.

6. Produit de synthèse d'acide nucléique selon la revendication 5, comprenant au moins deux régions d'acide nucléique exonique flanquant une région d'acide nucléique intronique, lequel est capable de coder ledit au moins un snoARN modifié.

7. Produit de synthèse d'acide nucléique selon la revendication 5 ou la revendication 6, où le produit de synthèse d'acide nucléique comprend une multiplicité de séquences exoniques flanquant deux séquences introniques ou plus comprenant une séquence capable de coder un ou plusieurs snoARN.

8. Cellule hôte transformée ou transfectée avec un produit de synthèse d'acide nucléique selon l'une ou l'autre des revendications 5-7.

9. Produit de synthèse de vecteur d'acide nucléique à utiliser pour créer des molécules de snoARN modifié, le produit de synthèse comprenant dans une direction de 5' en 3':
i) une séquence promoteur pour contrôler la transcription;
ii) une première séquence exon;
iii) une première séquence d'épissage d'introns;
iv) un site ou une séquence de clonage pour faciliter le clonage d'une séquence d'acide nucléique codant un snoARN modifié selon l'une quelconque des revendications 2-3;
v) une deuxième séquence d'épissage d'introns; et
vi) une deuxième séquence exon.

10. Produit de synthèse de vecteur d'acide nucléique selon la revendication 9, comprenant en outre dans une direction de 5' en 3':
vii) une troisième séquence ou séquence d'épissage supplémentaire d'introns;
vii) un deuxième site ou séquence ou site ou séquence de clonage supplémentaire pour faciliter le clonage d'une séquence d'acide nucléique codant un snoARN modifié selon l'une quelconque des revendications 2-3;
viii) une quatrième séquence ou séquence d'épissage supplémentaire d'introns; et
ix) une troisième séquence ou séquence exon supplémentaire.

11. Produit de synthèse de vecteur d'acide nucléique selon les revendications 5-7, 9 ou 10, comprenant en outre une séquence codante exprimable codant une protéine, un ARN, une protéine marquée, une protéine mutante ou ainsi de suite, pour remplacer fonctionnellement l'ARN cible ou la protéine à moduler par le(s)dit(s) un ou plusieurs snoARN.

12. SnoARN, snoARN modifié et/ou fragment de celui-ci, capable de s'hybrider à une portion d'acide nucléique cible, ou produit de synthèse d'acide nucléique codant un snoARN ou un snoARN modifié et/ou un fragment de celui-ci, capable de s'hybrider à une portion d'acide nucléique cible, ou cellule capable d'exprimer un snoARN ou un snoARN modifié et/ou un fragment de celui-ci, capable de s'hydrider à une portion d'acide nucléique cible, ou à utiliser dans le traitement d'une maladie ou d'une condition chez un sujet chez lequel il est désirable de moduler, particulièrement de réguler à la baisse l'expression d'un acide nucléique cible.

13. Composition pharmaceutique comprenant un snoARN, un snoARN modifié et/ou un fragment de celui-ci, capable de s'hybrider à une portion d'acide nucléique cible, ou un produit de synthèse d'acide nucléique codant un snoARN ou un snoARN modifié et/ou un fragment de celui-ci, capable de s'hybrider à une portion d'acide nucléique cible, ou une cellule capable d'exprimer un snoARN ou un snoARN modifié et/ou un fragment de celui-ci, capable de s'hybrider à une portion d'acide nucléique cible, et un véhicule pharmaceutiquement acceptable pour ceci.

14. Produit ou composition selon l'une ou l'autre des revendications 12 ou 13, comprenant un snoARN modifié, un produit de synthèse d'acide nucléique codant un snoARN ou un snoARN modifié, ou une cellule capable d'exprimer un snoARN ou un snoARN modifié, selon l'une quelconque des revendications 2-11.

15. Utilisation d'un vecteur selon les revendications 5-7, 9 ou 10, pour évaluer des phénotypes mutants, ou pour aider les stratégies de criblage de médicaments, ou pour la validation de cible, ou pour analyser une forme marquée d'une protéine sans compétition de la forme cellulaire existante, non marquée, ou pour d'autres mises en oeuvre appliquées ou de recherche.


### SEQUENCE LISTING

<110> the university Court of the university of Dundee
   LAMOND , ANGUS I
   ONO, MOTOHARU I
<120> TARGETED MODULATION OF GENE EXPRESSION
<130> PC/P15434PC
<160> 46
<170> PatentIn version 3.3
<210> 1
   <211> 97
   <212> DNA
   <213> Artificial
<220>
   <223> Plasmid sequences
<400> 1
   cuggggcucc caugaugucc agcacugggc ucugaucacc ccugaggaca cagugcaccc 60
   caggaccuuu gacaccuggg ggucugaggg gccccag 97
<210> 2
   <211> 97
   <212> DNA
   <213> Artificial
<220>
   <223> Plasmid sequences
<400> 2
   cuggggcucc caugaaaacc agcacugggc ucugaucacc ccugaggaca cagugcaccc 60
   caggaccuuu gacaccuggg ggucugaggg gccccag 97
<210> 3
   <211> 97
   <212> DNA
   <213> Artificial
<220>
   <223> Plasmid sequences
<400> 3
   cuggggcucc caugaugucc agcaaaaggc ucugaucacc ccugaggaca cagugcaccc 60
   caggaccuuu gacaccuggg ggucugaggg gccccag 97
<210> 4
   <211> 97
   <212> DNA
   <213> Artificial
<220>
   <223> Plasmid sequences
<400> 4
   cuggggcucc caugaugucc agcacugggc uaaaaucacc ccugaggaca cagugcaccc 60
   caggaccuuu gacaccuggg ggucugaggg gccccag 97
<210> 5
   <211> 97
   <212> DNA
   <213> Artificial
<220>
   <223> Plasmid sequences
<400> 5
   cuggggcucc caugaugucc agcacugggc ucugaucacc ccugaggaca cagugcaaaa 60
   caggaccuuu gacaccuggg ggucugaggg gccccag 97
<210> 6
   <211> 97
   <212> DNA
   <213> Artificial
<220>
   <223> Plasmid sequences
<400> 6
   cuggggcucc caugaugucc agcacugggc ucugaucacc ccugaggaca cagugcaccc 60
   caggaaaauu gacaccuggg ggucugaggg gccccag 97
<210> 7
   <211> 97
   <212> DNA
   <213> Artificial
<220>
   <223> Plasmid sequences
<400> 7
   cuggggcucc caugaugucc agcacugggc ucugaucacc ccugaggaca cagugcaccc 60
   caggaccuuu gacaccuaaa ggucugaggg gccccag 97
<210> 8
   <211> 97
   <212> DNA
   <213> Artificial
<220>
   <223> Plasmid sequences
<400> 8
   cuggggcucc caugaugucc agcacugggc ucugaucacc ccugaggaca cagugcaccc 60
   caggaccuuu gacaccuggg gguaaaaggg gccccag 97
<210> 9
   <211> 97
   <212> DNA
   <213> Artificial
<220>
   <223> Plasmid sequences
<400> 9
   cuggggcucc caugaugucc agcacugggc ucugaucacc ccugaggaca cagugcaccc 60
   caggaccuuu gacaccuggg ggucugagaa accccag 97
<210> 10
   <211> 97
   <212> DNA
   <213> Artificial
<220>
   <223> Plasmid sequences
<400> 10
   cuggggcucc caugaugucc agcacugggc ucugauaccu ugaugccguu cuucugcccc 60
   caggaccuuu gacaccuggg ggucugaggg gccccag 97
<210> 11
   <211> 97
   <212> DNA
   <213> Artificial
<220>
   <223> Plasmid sequences
<400> 11
   cuggggcucc caugaaaacc agcacugggc ucugauaccu ugaugccguu cuucugcccc 60
   caggaccuuu gacaccuggg ggucugaggg gccccag 97
<210> 12
   <211> 97
   <212> DNA
   <213> Artificial
<220>
   <223> Plasmid sequences
<400> 12
   cuggggcucc caugaugucc agcacugggc ucugauaccu ugaugccguu cuucugcccc 60
   caggaccuuu gacaccuggg gguaaaaggg gccccag 97
<210> 13
   <211> 97
   <212> DNA
   <213> Artificial
<220>
   <223> Plasmid sequences
<400> 13
   cuggggcucc caugaugucc agcaaaaggc ucugauaccu ugaugccguu cuucugcccc 60
   caggaccuuu gacaccuggg ggucugaggg gccccag 97
<210> 14
   <211> 97
   <212> DNA
   <213> Artificial
<220>
   <223> Plasmid sequences
<400> 14
   cuggggcucc caugaugucc uuuuaaauuu ucugauaccu ugaugccguu cuucugcccc 60
   caggaccuuu gacaccuggg ggucugaggg gccccag 97
<210> 15
   <211> 97
   <212> DNA
   <213> Artificial
<220>
   <223> Plasmid sequences
<400> 15
   cuggggcucc caugaugucc agcacugggc uaaaauaccu ugaugccguu cuucugcccc 60
   caggaccuuu gacaccuggg ggucugaggg gccccag 97
<210> 16
   <211> 99
   <212> DNA
   <213> Artificial
<220>
   <223> Plasmid sequences
<400> 16
   cuggggcucc caugaugucc agcacugggc ucugauucac cuugaugccg uucuucugcc 60
   cccaggaccu uugacaccug ggggucugag gggccccag 99
<210> 17
   <211> 101
   <212> DNA
   <213> Artificial
<220>
   <223> Plasmid sequences
<400> 17
   cuggggcucc caugaugucc agcacugggc ucugauucac cuugaugccg uucuucuguu 60
   cccccaggac cuuugacacc ugggggucug aggggcccca g 101
<210> 18
   <211> 105
   <212> DNA
   <213> Artificial
<220>
   <223> Plasmid sequences
<400> 18
   cuggggcucc caugaugucc agcacugggc ucugauguuc accuugaugc cguucuucug 60
   uugtccccca ggaccuuuga caccuggggg ucugaggggc cccag 105
<210> 19
   <211> 95
   <212> DNA
   <213> Artificial
<220>
   <223> Plasmid sequences
<400> 19
   cuggggcucc caugaugucc agcacugggc ucugaucuug augccguucu ucugccccca 60
   ggaccuuuga caccuggggg ucugaggggc cccag 95
<210> 20
   <211> 94
   <212> DNA
   <213> Artificial
<220>
   <223> Plasmid sequences
<400> 20
   cuggggcucc caugaugucc agcacugggc ucugaucuug augccguucu ucucccccag 60
   gaccuuugac accugggggu cugaggggcc ccag 94
<210> 21
   <211> 90
   <212> DNA
   <213> Artificial
<220>
   <223> Plasmid sequences
<400> 21
   cuggggcucc caugaugucc agcacugggc ucugauugau gccguucuuc ccccaggacc 60
   uuugacaccu gggggucuga ggggccccag 90
<210> 22
   <211> 97
   <212> DNA
   <213> Artificial
<220>
   <223> Plasmid sequences
<400> 22
   cuggggcucc caugaugucc agcacugggc ucugauaccu ugauaccguu cuucugcccc 60
   caggaccuuu gacaccuggg ggucugaggg gccccag 97
<210> 23
   <211> 97
   <212> DNA
   <213> Artificial
<220>
   <223> Plasmid sequences
<400> 23
   cuggggcucc caugaugucc agcacugggc ucugauaccu ugauacaguu cuucugcccc 60
   caggaccuuu gacaccuggg ggucugaggg gccccag 97
<210> 24
   <211> 97
   <212> DNA
   <213> Artificial
<220>
   <223> Plasmid sequences
<400> 24
   cuggggcucc caugaugucc agcacugggc ucugauaccu ugauacaguu auucugcccc 60
   caggaccuuu gacaccuggg ggucugaggg gccccag 97
<210> 25
   <211> 97
   <212> DNA
   <213> Artificial
<220>
   <223> Plasmid sequences
<400> 25
   cuggggcucc caugaugucc agcacugggc ucugauaccu uaauacaguu auucugcccc 60
   caggaccuuu gacaccuggg ggucugaggg gccccag 97
<210> 26
   <211> 97
   <212> DNA
   <213> Artificial
<220>
   <223> Plasmid sequences
<400> 26
   cuggggcucc caugaugucc agcacugggc ucugauaccu uaauacaguu auuaugcccc 60
   caggaccuuu gacaccuggg ggucugaggg gccccag 97
<210> 27
   <211> 97
   <212> RNA
   <213> Artificial
<220>
   <223> Plasmid sequences
<400> 27
   cuggggcucc caugaugucc agcacugggc ucugauacau uaauacaguu auuaugcccc 60
   caggaccuuu gacaccuggg ggucugaggg gccccag 97
<210> 28
   <211> 21
   <212> DNA
   <213> Artificial
<220>
   <223> Plasmid sequences
<400> 28
   caccccagag gacacagtgc a 21
<210> 29
   <211> 21
   <212> DNA
   <213> Artificial
<220>
   <223> Plasmid sequences
<400> 29
   gacttgaaga agtcgtgctg c 21
<210> 30
   <211> 21
   <212> DNA
   <213> Artificial
<220>
   <223> Plasmid sequences
<400> 30
   accttgatgc cgttcttctg c 21
<210> 31
   <211> 40
   <212> DNA
   <213> Artificial
<220>
   <223> probe
<400> 31
   cactcagacc gcgttctctc cctctcactc cccaatacgg 40
<210> 32
   <211> 40
   <212> DNA
   <213> Artificial
<220>
   <223> probe
<400> 32
   aaaggtcctg gggtgcactg tgtcctcagg ggtgatcaga 40
<210> 33
   <211> 40
   <212> DNA
   <213> Artificial
<220>
   <223> probe
<400> 33
   acgacggtat gagttctcac tcattttgtt cagcttttcc 40
<210> 34
   <211> 24
   <212> DNA
   <213> Artificial
<220>
   <223> primer
<400> 34
   agaggtagcg ttttctcctg agcg 24
<210> 35
   <211> 20
   <212> DNA
   <213> Artificial
<220>
   <223> primer
<400> 35 20
   accactcaga ccgcgttctc 20
<210> 36
   <211> 20
   <212> DNA
   <213> Artificial
<220>
   <223> primer
<400> 36 20
   ctcccatgat gtccagcact 20
<210> 37
   <211> 20
   <212> DNA
   <213> Artificial
<220>
   <223> primer
<400> 37 20
   ctcagacccc caggtgtcaa 20
<210> 38
   <211> 27
   <212> DNA
   <213> Artificial
<220>
   <223> primer
<400> 38
   gatcgatgat gagtccccca taaaaac 27
<210> 39
   <211> 26
   <212> DNA
   <213> Artificial
<220>
   <223> primer
<400> 39 26
   gacctcagtt ctgatgagaa cgacgg 26
<210> 40
   <211> 24
   <212> DNA
   <213> Artificial
<220>
   <223> primer
<400> 40
   tggacgtgct ggagcgctcc ccgc 24
<210> 41
   <211> 26
   <212> DNA
   <213> Artificial
<220> ,
   <223> primer
<400> 41
   cccagggtca gccgggcccg agacag 26
<210> 42
   <211> 23
   <212> DNA
   <213> Artificial
<220>
   <223> primer
<400> 42
   cgcatcttct tttgcgtcgc cag 23
<210> 43
   <211> 25
   <212> DNA
   <213> Artificial
<220>
   <223> primer
<400> 43
   ggtcaatgaa ggggtcattg atggc 25
<210> 44
   <211> 21
   <212> DNA
   <213> Artificial
<220>
   <223> Plasmid sequence
<400> 44
   gacttgaaga agtcgtgctg c 21
<210> 45
   <211> 21
   <212> DNA
   <213> Artificial
<220>
   <223> Plasmid sequence
<400> 45
   accttgatgc cgttcttctg c 21
<210> 46
   <211> 21
   <212> DNA
   <213> Artificial
<220>
   <223> Plasmid sequence
<400> 46
   cacccctgag gacacagtgc a 21